# EUROPEAN PATENT SPECIFICATION

(11) **EP 3 164 171 B1**
(45) Date of publication and mention of the grant of the patent: **11.05.2022**
(21) Application number: 15745615.3
(22) Date of filing: 02.07.2015
(51) Int. Cl.: A61M 5/168, A61M 39/08, A61M 39/10, A61M 5/142, A61M 39/00, A61M 39/02

(54) **INFUSION PUMP ASSEMBLY**
INFUSIONSPUMPENANORDNUNG
ENSEMBLE POMPE D'INFUSION

(30) Priority: 03.07.2014 US 201414323571; 03.07.2014 US 201414323604
(43) Date of publication of application: 10.05.2017
(73) Proprietor: DEKA Products Limited Partnership, Manchester, NH 03101-1129 (US)
(72) Inventor: GRANT, Kevin, L., Litchfield, NH 03052 (US); DEMERS, Jason, A., Manchester, NH 03104 (US); TRACEY, Brian, D., Litchfield, NH 03052 (US); KAMEN, Dean, Bedford, NH 03110 (US); LANIGAN, Richard, J., Concord, NH 03301 (US); LANIER, Gregory, R., Merrimack, NH 03054 (US); FOO, Bright, C.k, Hollis, NH 03049 (US); GUSTIN, Lisa, A., Manchester, NH 03102 (US); SOLDAU, Thomas, F., Bedford, NH 03110 (US); FICHERA, Stephen, L., Salem, NH 03079 (US); CANNAN, David, D.B., Manchester, NH 03104 (US); MOREAU, Timothy, D., Manchester, NH 03104 (US)
(74) Representative: Elkington and Fife LLP
(86) International application number: PCT/US2015/039026
(87) International publication number: WO 2016/004329

(56) References cited:
- WO-A2-2013/134486

## Description

### FIELD OF THE INVENTION

This application relates generally to fluid delivery systems, and more particularly to infusion pump assemblies.

### BACKGROUND

Many potentially valuable medicines or compounds, including biologicals, are not orally active due to poor absorption, hepatic metabolism or other pharmacokinetic factors. Additionally, some therapeutic compounds, although they can be orally absorbed, are sometimes required to be administered so often it is difficult for a patient to maintain the desired schedule. In these cases, parenteral delivery is often employed or could be employed.

Effective parenteral routes of drug delivery, as well as other fluids and compounds, such as subcutaneous injection, intramuscular injection, and intravenous (IV) administration include puncture of the skin with a needle or stylet. Insulin is an example of a therapeutic fluid that is self-injected by millions of diabetic patients. Users of parenterally delivered drugs may benefit from a wearable device that would automatically deliver needed drugs/compounds over a period of time.

To this end, there have been efforts to design portable and wearable devices for the controlled release of therapeutics. Such devices are known to have a reservoir such as a cartridge, syringe, or bag, and to be electronically controlled. These devices suffer from a number of drawbacks including the malfunction rate. Reducing the size, weight and cost of these devices is also an ongoing challenge. Additionally, these devices often apply to the skin and pose the challenge of frequent re-location for application. WO2013/134486 discloses an infusion pump system comprising a fluid connector assembly.

### SUMMARY OF THE INVENTION

In accordance with an aspect of the invention, there is provided an infusion pump system according to claim 1. The infusion pump system includes a reusable housing assembly comprising a pump actuator assembly; a disposable housing assembly including a reservoir; a tab portion, the tab portion comprising a female latching feature; and an exit, the exit fluidly connected to the reservoir; and
a fluid connector including a body portion comprising a slot; a plug portion slidably connected to the body portion, the plug portion comprising a fluid path and a disc, the disc configured to seat within the slot; a catch feature located on a first end of the body portion and configured to interact with the disposable housing assembly; a male latching feature located on a second end of the body portion, the male latching feature configured to interact and lock onto the female latching feature; and a tubing, a first end of the tubing fluidly connected to the plug fluid path; wherein force applied to the plug portion may overcome a threshold force and unseat the disc from the slot wherein the plug portion moves with respect to the body portion, wherein the plug of the connector forms a luer connection with the exit of the disposable housing assembly and provides a fluid connection between the reservoir and the tubing, wherein the reusable housing assembly may be attached to the disposable housing assembly, and wherein the pump actuator assembly pumps fluid from the reservoir to the tubing.

Some embodiments of this implementation may include one or more of the following features. Wherein the reusable housing assembly further comprising a volume sensor assembly.
Wherein the second end of the tubing connected to a cannula assembly. Wherein the connector further comprising wherein the body portion comprising an indent wherein the indent configured to interact with a reusable portion of an infusion pump. Wherein the reusable housing assembly further comprising a locking ring assembly. Wherein the locking ring assembly further comprising a nub comprising a spring actuated tab, the spring actuated tab interacts with the indent of the connector when the reusable housing assembly and disposable housing assembly are rotatably attached. Wherein the spring actuated tab of the nub interacts with the connector exerting compression onto the connector attachment to the exit of the disposable housing assembly. Wherein the body portion further comprising rib features. Wherein the catch feature comprising a ramp. Wherein the underside of the body portion comprising a core. Wherein the core comprising an identification tag. Wherein the body portion comprising an identification tag.
Wherein the identification tag is an RFID tag. Wherein the identification tag is a near-field communication readable RFID.

In accordance with one example, a fluid reservoir system is disclosed. The fluid reservoir system includes a disposable housing assembly including a reservoir; a tab portion, the tab portion comprising a female latching feature; and an exit, the exit fluidly connected to the reservoir; and a connector comprising: a body portion comprising a slot; a plug portion slidably connected to the body portion, the plug portion comprising a fluid path and a disc, the disc configured to seat within the slot; a catch feature located on a first end of the body portion and configured to interact with the disposable housing assembly; and a male latching feature located on a second end of the body portion, the male latching feature configured to interact and lock onto the female latching feature, wherein the plug of the connector attaches to the exit of the disposable housing assembly and provides a fluid connection between the reservoir and the tubing, and wherein force applied to the plug portion may overcome a threshold force and unseat the disc from the slot wherein the plug portion moves with respect to the body portion.

Some examples may include one or more of the following features. Wherein the connector further includes wherein the body portion comprising an indent wherein the indent configured to interact with a reusable portion of an infusion pump. Wherein the body portion further comprising rib features. Wherein the catch feature comprising a ramp.
Wherein the first end of the body portion comprising an locked icon. Wherein the second end of the body portion comprising an unlocked icon. Wherein the body portion further comprising an arrow icon. Wherein the underside of the body portion comprising a core. Wherein the core comprising an identification tag. Wherein the body portion comprising an identification tag. Wherein the identification tag is an RFID tag. Wherein the identification tag is a near-field communication readable RFID.

In accordance with one example a fluid connector assembly is disclosed. The fluid connector assembly includes a tab portion including a slot; a plug portion slidably connected to the tab portion the plug portion comprising a fluid path and a disc, the disc configured to seat within the slot; a catch feature located on a first end of the tab portion and configured to interact with a reservoir; and a latching feature located on a second end of the tab portion, the latching feature configured to interact and lock onto the reservoir, wherein force applied to the plug portion may overcome a threshold force and unseat the disc from the slot wherein the plug portion moves with respect to the tab portion.

Some examples of this implementation may include one or more of the following features. Wherein the tab portion further comprising an indent wherein the indent configured to interact with a reusable housing assembly. Wherein the connector includes a tubing connected to the plug Wherein the catch feature comprising a ramp. Wherein the second end of the tubing connected to a cannula assembly. Wherein the tab portion further comprising a tapered tubing opening, the first end of the tubing connecting to the tapered tubing opening. Wherein the underside of the tab portion comprising a core. Wherein the core comprising an identification tag. Wherein the tab portion comprising an identification tag. Wherein the identification tag is an RFID tag. Wherein the identification tag is a near-field communication readable RFID.

In accordance with one example a fluid connector assembly is disclosed. The fluid connector assembly includes a fluid connector assembly including a tab portion including a tubing side and a reservoir side; a plug portion slidably connected to the tab portion, the plug portion comprising a fluid path; a catch feature located on a first end of the tab portion and configured to interact with a reservoir; and a latching feature located on a second end of the tab portion, the latching feature configured to interact and lock onto the reservoir, wherein force applied to the plug portion causes the plug portion to move with respect to the tab portion from an initial position to a final position in the direction of the tubing side of the tab portion.
Some examples of this implementation may include one or more of the following features. Wherein the tab portion further comprising an indent wherein the indent configured to interact with a reusable housing assembly. Wherein the connector includes a tubing connected to the plug Wherein the catch feature comprising a ramp. Wherein the second end of the tubing connected to a cannula assembly. Wherein the tab portion further comprising a tapered tubing opening, the first end of the tubing connecting to the tapered tubing opening. Wherein the underside of the tab portion comprising a core. Wherein the core comprising an identification tag. Wherein the tab portion comprising an identification tag. Wherein the identification tag is an RFID tag. Wherein the identification tag is a near-field communication readable RFID.

In accordance with one example, a connector. The connector includes a body portion, a plug, and a tubing in communication with the plug, wherein the plug is configured to attach to an exit in a disposable housing assembly.

In accordance with first example, a wearable infusion pump assembly is disclosed. The wearable infusion pump assembly includes a reservoir for receiving an infusible fluid and a fluid delivery system configured to deliver the infusible fluid from the reservoir to an external infusion set. The fluid delivery system includes a controller, a pump assembly for extracting a quantity of infusible fluid from the reservoir and providing the quantity of infusible fluid to the external infusion set, the pump assembly comprising a pump plunger, the pump plunger having distance of travel, the distance of travel having a starting position and an ending position, at least one optical sensor assembly for sensing the starting position and ending position of the pump plunger distance of travel and sending sensor output to the controller, and a first valve assembly configured to selectively isolate the pump assembly from the reservoir, wherein the controller receives the sensor output and determines the total displacement of the pump plunger.

Some embodiments of this example may include one or more of the following features. Wherein the wearable infusion pump assembly includes wherein the controller correlates the displacement of the pump plunger to a volume of fluid delivered. Wherein the wearable infusion pump assembly includes wherein the controller, based on the volume of fluid delivered, commands an actuator to actuate the pump plunger to a target position. Wherein the wearable infusion pump assembly further includes a second valve assembly configured to selectively isolate the pump assembly from the external infusion set. Wherein the wearable infusion pump assembly further includes at least one optical sensor assembly for sensing the position of the second valve assembly. Wherein the wearable infusion pump assembly further includes a disposable housing assembly including the reservoir and a first portion of the fluid delivery system, and a reusable housing assembly including a second portion of the fluid delivery system. Wherein the wearable infusion pump assembly includes wherein a first portion of the pump assembly is positioned within the disposable housing assembly, and a second portion of the pump assembly is positioned within the reusable housing assembly. Wherein the wearable infusion pump assembly includes wherein a first portion of the first valve assembly is positioned within the disposable housing assembly, and a second portion of the first valve assembly is positioned within the reusable housing assembly. Wherein the wearable infusion pump assembly includes wherein a first portion of the second valve assembly is positioned within the disposable housing assembly, and a second portion of the second valve assembly is positioned within the reusable housing assembly. Wherein the wearable infusion pump assembly includes wherein the external infusion set is a detachable external infusion set configured to releasably engage the fluid delivery system.

In accordance with first example, a disposable housing assembly for an infusion pump assembly is disclosed. The disposable housing assembly includes a reservoir portion fluidly connected to a fluid path, the reservoir portion including a bubble trap wherein the bubble trap prevents air from moving from the reservoir portion to the fluid path. The bubble trap further includes an outlet portion and a non-outlet portion, the non-outlet portion including a tapered portion that tapers to a bottom portion, the tapered portion of the non-outlet portion ending at the outlet portion. The bubble trap also includes wherein the outlet portion including the bottom portion in communication with an upward ramped portion in fluid communication with a reservoir outlet, wherein the bottom portion configured whereby fluid congregates in the bottom portion and the tapered portion configured whereby air bubbles congregate in the tapered portion.

Some embodiments of this example may include one or more of the following features. Wherein the disposable housing assembly further includes a membrane assembly, the membrane assembly connected to the reservoir wherein the membrane assembly forms a portion of the reservoir. Wherein the disposable housing assembly further includes a septum assembly, the septum assembly formed on the membrane assembly. Wherein the disposable housing assembly further includes a septum assembly, the septum assembly connected to the reservoir. Wherein the disposable housing assembly further includes a vent, wherein the vent further comprising a filter.

In accordance with one example, a fluid connector assembly is disclosed. The fluid connector assembly includes a body portion, a plug receiver portion located on the body portion, the plug receiver portion including a fluid path and configured to receive a plug on a reservoir, and a tubing, a first end of the tubing fluidly connected to the plug receiver portion fluid path.

Some embodiments of this example may include one or more of the following features. Wherein the body portion further includes an indent wherein the indent configured to interact with a reusable portion of an infusion pump. Wherein a second end of the tubing connected to a cannula assembly. Wherein the body portion further includes a tapered tubing opening, the first end of the tubing connecting to the tapered tubing opening. Wherein a first end of the body portion further comprising a locked icon. Wherein the underside of the body portion comprising a core. Wherein the core comprising an identification tag. Wherein the body portion comprising an identification tag. Wherein the identification tag is an RFID tag. Wherein the identification tag is a near-field communication readable RFID.

In accordance with one example, a clip assembly is disclosed. The clip assembly includes a clip portion and a clip housing portion. The clip housing portion includes a front portion and a back portion. The back portion includes at least one protrusion and at least one clip feature, wherein the at least one clip feature secures the clip portion onto the back portion of the housing portion.
In accordance with one example, a filling aid is disclosed. The filling aid includes a locking portion including a groove feature; and a locking feature having a locked and an unlocked position; and a filling syringe holder slidably attached to the locking portion, the filling syringe holder including a filling needle cradle portion having a tongue feature; and a needle housing portion comprising at least one tab having a starting position and a filling position,wherein the groove feature configured to accommodate the tongue feature, and
wherein the locking feature interact with the filling syringe holder wherein when the locking feature moves from the locked position to the unlocked position the needle housing portion moves from the starting position to the filling position.

Some s examples include one or more of the following. Wherein when the locking feature moves from the unlocked position to the locked position the needle housing portion moves from the filling position to the starting position. Wherein the filling need cradle portion further comprising a frame portion whereby the frame portion maintains a filling syringe in fixed relation with the filling syringe holder. Wherein the filling syringe holder further comprising grips. Wherein the grips comprising gripping features. Wherein when the locking feature attaches to a filling aid base the locking feature moves from a locked position to an unlocked position. Wherein when the locking feature moves from a locked position to an unlocked position, the filling syringe holder slides towards the filling aid base. Wherein the filling needle cradle portion further comprising filling needle cradle ribs which capture a filling syringe and maintain the filling syringe in an attached position with respect to the filling needle cradle. Wherein the filling needle cradle portion further comprising a stop feature that limits the distance the filling syringe may travel within the filling needle cradle portion. Wherein the frame portion comprising a half moon portion that accommodates a sliding portion of a filling syringe and provides a stop for the filling syringe. Wherein the locking portion further comprising at least one tab feature. Wherein the locking portion further comprising a filling syringe support that maintains the position of the filling syringe with respect to the filling aid.

In accordance with one example, a system for filling a reservoir is disclosed. The system includes a disposable housing assembly comprising a reservoir; a fill adapter for releasably engaging the disposable housing assembly; and a filling aid configured to releasably engage the fill adapter, the filling aid including a locking portion including a groove feature; and a locking feature having a locked and an unlocked position; and a filling syringe holder slidably attached to the locking portion, the filling syringe holder including a filling needle cradle portion having a tongue feature; and a needle housing portion comprising at least one tab having a starting position and a filling position, wherein the groove feature configured to accommodate the tongue feature, and wherein the locking feature interact with the filling syringe holder wherein when the locking feature moves from the locked position to the unlocked position the needle housing portion moves from the starting position to the filling position.

Some examples include one or more of the following.
Wherein the filling need cradle portion further comprising a frame portion whereby the frame portion maintains a filling syringe in fixed relation with the filling syringe holder. Wherein when the locking feature attaches to a filling aid base the locking feature moves from a locked position to an unlocked position. Wherein when the locking feature moves from a locked position to an unlocked position, the filling syringe holder slides towards the filling aid base. Wherein the filling needle cradle portion further comprising filling needle cradle ribs which capture a filling syringe and maintain the filling syringe in an attached position with respect to the filling needle cradle. Wherein the filling needle cradle portion further comprising a stop feature that limits the distance the filling syringe may travel within the filling needle cradle portion. Wherein the system further comprising a filling syringe.

In accordance with one example, a filling aid is disclosed. The filling aid includes a locking portion comprising at least one locking feature and a filling syringe holder slidably connected to the locking portion and having having a starting position and a filling position with respect to the locking portion.

In accordance with one example, a filling aid is disclosed. The filling aid includes a needle housing portion comprising at least locking feature and having a starting position and a filling position, and a filling needle cradle comprising at least one cradle rib and a stop feature, the filling needle cradle slidably connected to the needle housing portion and having a starting position and a filling position.

In accordance with one example a fill adapter for filling a reservoir is disclosed. The fill adapter includes a housing having a cavity portion configured to attach to a reservoir, a filling aid base connected to the housing configured to receive a filling aid, a button assembly actuator springingly attached to the housing, the button assembly actuator comprising one or more button assemblies, and a pump chamber plunger actuator located in the cavity portion of the housing, wherein the pump chamber plunger actuator actuates a pump chamber membrane in the reservoir before the at least one button assembly is actuated.

Some examples include one or more of the following.
Wherein the fill adapter further includes a mechanism for locking a reservoir into the cavity portion. Wherein the fill adapter further includes at least one tab for releasably locking a reservoir into the cavity portion. Wherein at least one tab further comprising a ramping portion. Wherein the filling aid base includes a cylindrical structure comprising an opening to the cavity portion. Wherein the filling aid base further includes a groove at the base of the filling aid base.
Wherein the filling aid base further including a tongue feature.

In accordance with one example a filling aid is disclosed. The filling aid includes a needle housing portion including at least one tab having a starting position and a filling position, and a filling needle cradle including a filling needle, the filling needle cradle slidable connected to the needle housing portion and having a starting position and a filling position, wherein when the at least one tab on the needle housing moves from a starting position to a filling position, the filling needle cradle slides from a starting position to a filling position, and wherein when the at least one tab on the needle housing moves from a filling position to a starting position, the filling needle cradle slides from a filling position to a starting position.

Some examples o include one or more of the following. Wherein the needle housing portion further includes at least one groove feature. Wherein the filling needle cradle includes at least one orientation bar that fits into the groove feature of the needle housing whereby allowing the filling needle cradle to slide with respect to the needle housing. Wherein the filling needle cradle includes a connector for a filling syringe. Wherein the connector for a filling syringe includes a luer connector. Wherein the filling needle cradle includes a filling needle.

In accordance with one example a system for filling a reservoir is disclosed. The system includes a disposable housing assembly including a reservoir, a fill adapter for releasably engaging the disposable housing assembly, the fill adapter including a pump chamber plunger, and a filling aid configured to releasably engage the fill adapter, wherein the pump chamber plunger actuates a pump chamber in the disposable housing assembly when the disposable housing assembly and the fill adapter are engaged.

Some examples include one or more of the following. Wherein the filling aid includes a needle housing portion including at least one tab having a starting position and a filling position, and a filling needle cradle comprising a filling needle, the filling needle cradle slidable connected to the needle housing portion and having a starting position and a filling position, wherein when the at least one tab on the needle housing moves from a starting position to a filling position, the filling needle cradle slides from a starting position to a filling position. Wherein the fill adapter including a housing having a cavity portion configured to attach to the disposable housing assembly, a filling aid base connected to the housing configured to receive the filling aid and a button assembly actuator springingly attached to the housing, the button assembly actuator comprising one or more button assemblies.
A filling syringe, the filling syringe configured to connect to the filling aid. Wherein the filling needle cradle further includes a connector for a filling syringe.

In accordance with one example a fill adapter for filling a reservoir is disclosed. The fill adapter includes a button assembly actuator and a pump chamber plunger actuator hingably attached to the button assembly actuator, wherein the actuation of the button assembly actuator actuates the pump chamber plunger actuator and wherein the pump chamber plunger actuator actuates a pump chamber membrane before the at least one button assembly is actuated.

Some examples include one or more of the following.
Where the fill adapter further includes a filling aid wherein said filling aid is attached to the fill adapter base and wherein the filling aid accommodates a syringe. Wherein the fill adapter is adapter to connectably attach to a reservoir assembly wherein upon attachment, the reservoir assembly may be filled by a syringe. Wherein the button assembly further comprising at least one button assembly. Wherein the filling aid is removably attached to the fill adapter.

In accordance with one example a fill adapter base is disclosed. The fill adapter base includes a button assembly actuator for actuating at least one button assembly and a pump chamber plunger actuator hingably attached to the button assembly actuator wherein the actuation of the button assembly actuator actuates the pump chamber plunger actuator and wherein the pump chamber plunger actuator actuates a pump chamber membrane before the at least one button assembly is actuated.

Some examples include one or more of the following.
Wherein the fill adapter base further includes a filling aid wherein said filling aid is attached to the fill adapter base. Wherein the filling aid is removably attached to the fill adapter base. Wherein the filling aid is hingably attached to the fill adapter base. Wherein the pump chamber actuator is hingably attached to the button assembly actuator through a living hinge. Wherein the pump chamber actuator is hingably attached to the button assembly actuator through a pivot hinge. Wherein the button assembly actuator actuates at least three button assemblies.

In accordance with one example a fill adapter system for filling a reservoir is disclosed. The fill adapter system includes a fill adapter base includes a button assembly actuator for actuating at least one button assembly and a pump chamber plunger actuator hingably attached to the button assembly actuator, wherein the actuation of the button assembly actuator actuates the pump chamber plunger actuator and wherein the pump chamber plunger actuator actuates a pump chamber membrane before the at least one button assembly is actuated. The fill adapter system also includes a filling aid attached to the fill adapter base for accommodating a syringe for filling a reservoir.

Some examples include one or more of the following.
Wherein the filing aid is removably attached to the fill adapter base. Wherein the filling aid is hingably attached to the fill adapter base. Wherein the pump chamber actuator is hingably attached to the button assembly actuator through a living hinge. Wherein the pump chamber actuator is hingably attached to the button assembly actuator through a pivot hinge. Wherein the button assembly actuator actuates at least three button assemblies.

In accordance with one example a fill adapter system is disclosed. The fill adapter system includes a disposable housing assembly comprising a reservoir for holding fluid and a fill adapter including a button assembly actuator for actuating at least one button assembly, a pump chamber plunger actuator hingably attached to the button assembly actuator, wherein the actuation of the button assembly actuator actuates the pump chamber plunger actuator and wherein the pump chamber plunger actuator actuates a pump chamber membrane before the at least one button assembly is actuated, and a filling aid attached to the fill adapter base for accommodating a syringe for filling a reservoir.

In some examples the fill adapter system further includes a syringe for filling the reservoir of the disposable housing assembly and wherein the filling aid accommodates the syringe.

In accordance with another example, a fill adapter for filling a reservoir is disclosed. The fill adapter includes a button assembly actuator, a pump chamber plunger actuator hingably attached to the button assembly actuator, wherein the actuation of the button assembly actuator actuates the pump chamber plunger actuator and wherein the pump chamber plunger actuator actuates a pump chamber membrane before the at least one button assembly is actuated, and a filling aid adapted to connect to the fill adapter, the filling aid comprising a filling needle assembly slidably connected to the inside of the filling aid.

The details of one or more embodiments are set forth in the accompanying drawings and the description below. Other features and advantages will become apparent from the description, the drawings, and the claims.

### BRIEF DESCRIPTION OF THE DRAWINGS

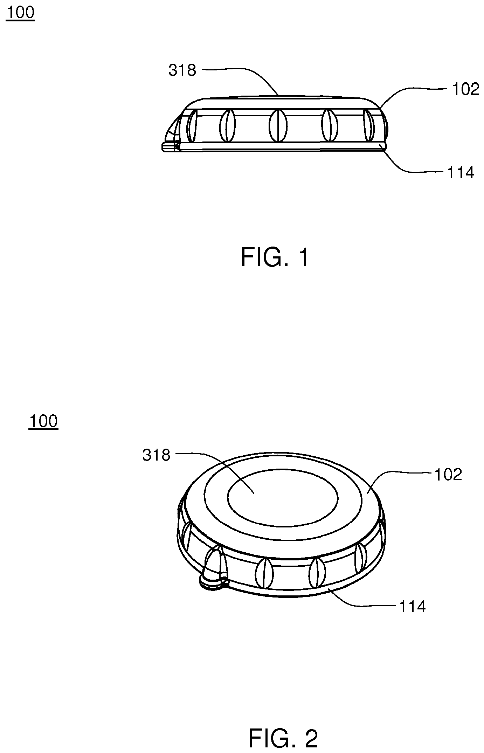

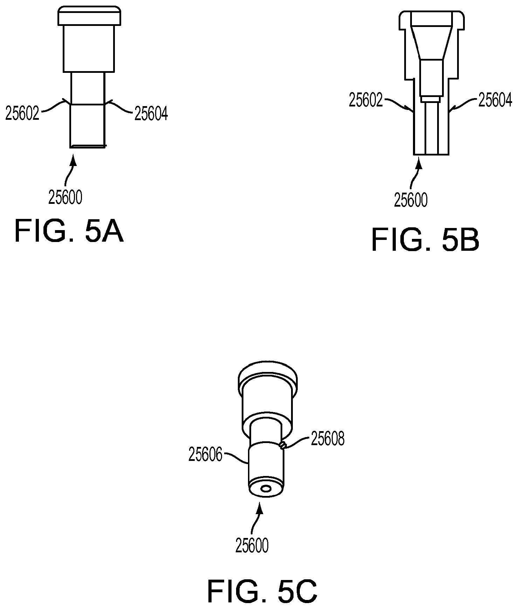

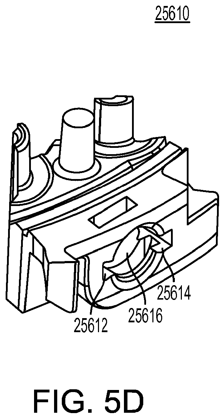

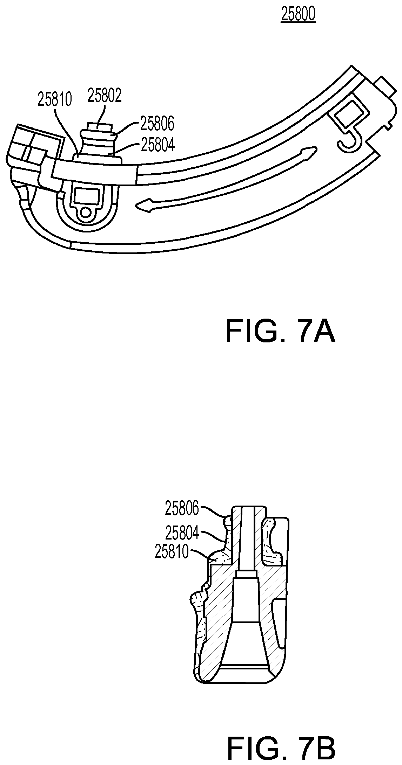

Figures 1-4 illustrate an infusion pump assembly that does not include all the features of the invention as presently claimed but represents subject-matter useful for understanding the invention. Figure 5A-C do not illustrate subject-matter including all the features of the invention as presently claimed, but illustrate a plug portion according to embodiments of the invention as presently claimed. Figures 7-13 do not illustrate subject-matter including all the features of the invention as presently claimed, but illustrate a connector and disposable housing assembly according to embodiments of the invention as presently claimed. Figures 5D and 14-29C do not illustrate subject-matter including all the features of the invention as presently claimed, but illustrate subject-matter useful for understanding the invention. FIG. 1 is a side view of an infusion pump assembly;
FIG. 2 is a perspective view of the infusion pump assembly of FIG. 1;
FIG. 3 is an exploded view of various components of the infusion pump assembly of FIG. 1;
FIG. 4 is a cross-sectional view of the disposable housing assembly of the infusion pump assembly of FIG. 1;
FIGS. 5A-5C are various views of examples of a plug according to an embodiment of the invention;
FIG. 5D is a partial view of one example of the exit of a disposable housing assembly;
FIG. 6A is a view of one examples of a connector;
FIG. 6B is a cross sectional view of one example of the plug shown in FIG. 6A;
FIG. 7A is a view of one example of a connector;
FIG. 7B is a cross sectional view of one example of the plug shown in FIG. 7A;
FIG. 8A is a view of one example of a connector;

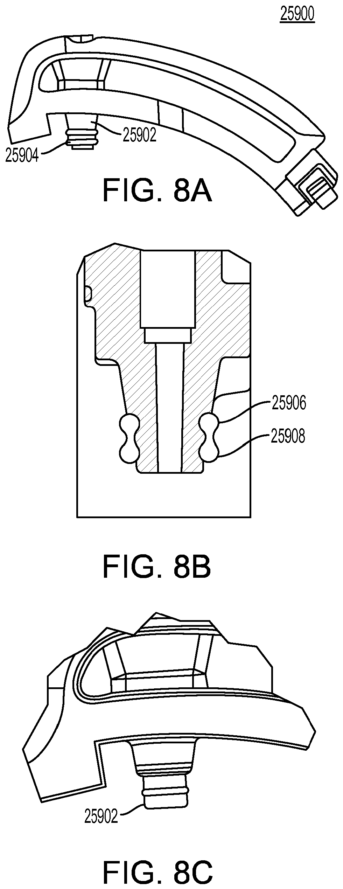

FIG. 7B is a cross sectional view of one example of the plug shown in FIG. 8A;
FIG. 8C is a partial view of an example of a connector;

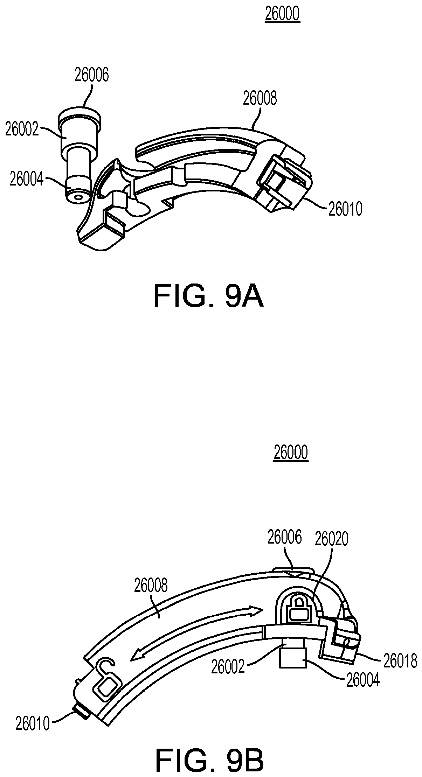

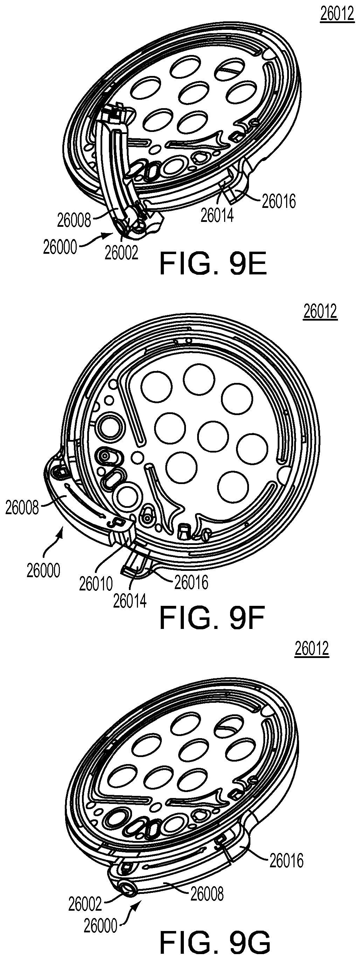

FIGS. 9A-9G are various views of one example of a connector;

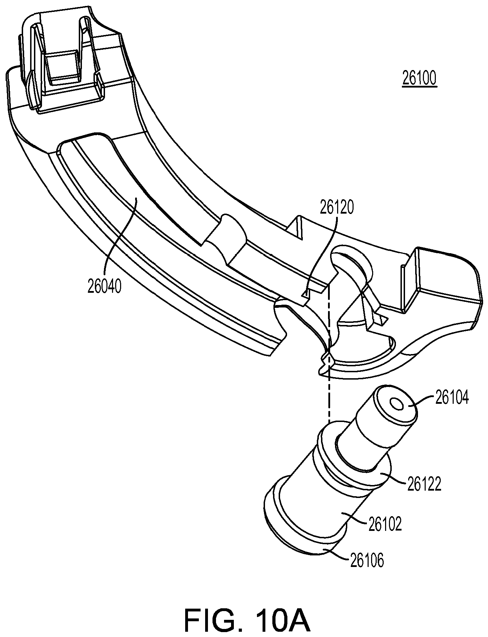

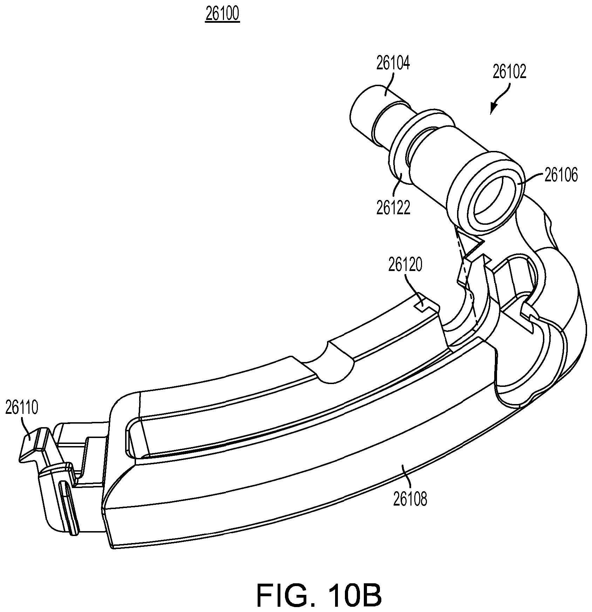

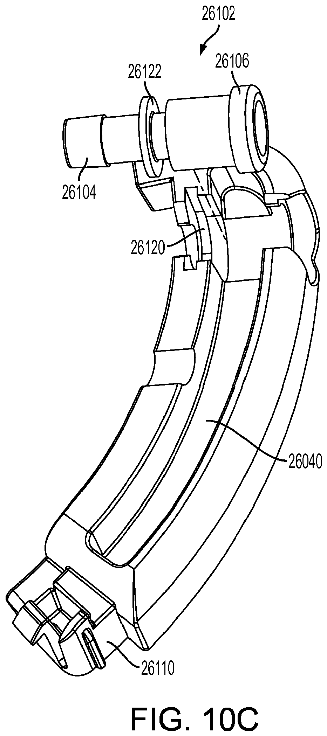

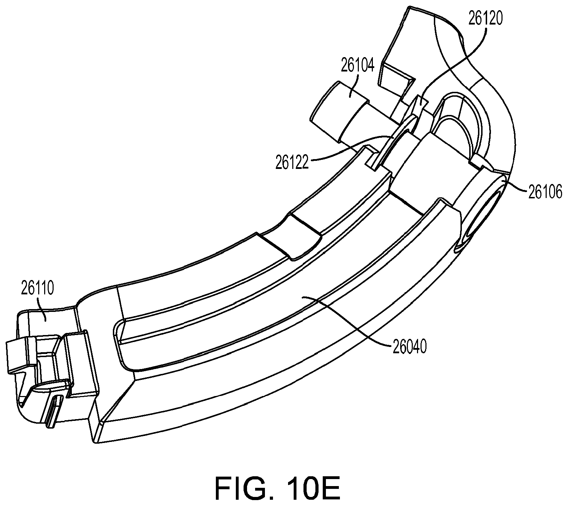

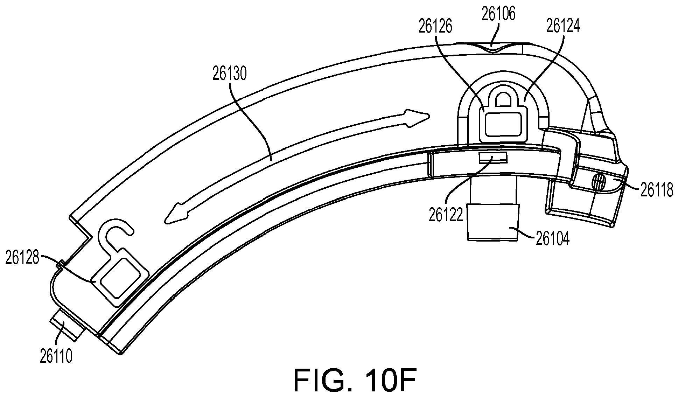

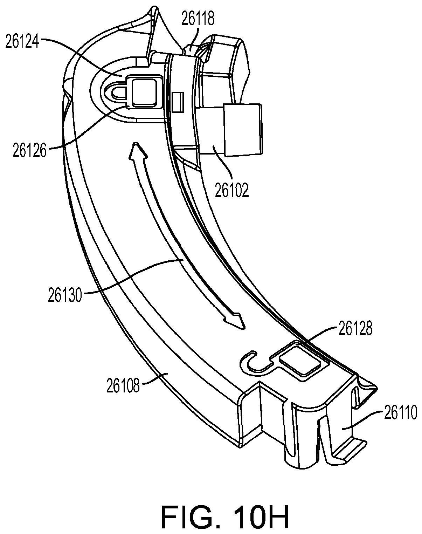

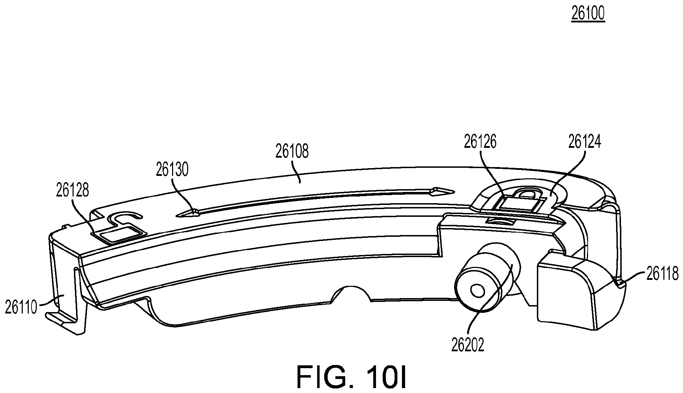

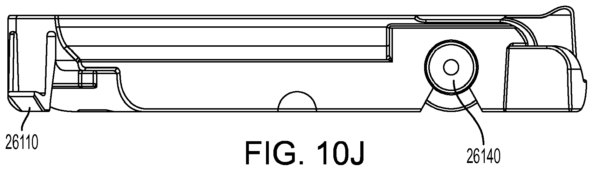

FIGS. 10A-11K are various views of a connector according to an embodiment of the invention;

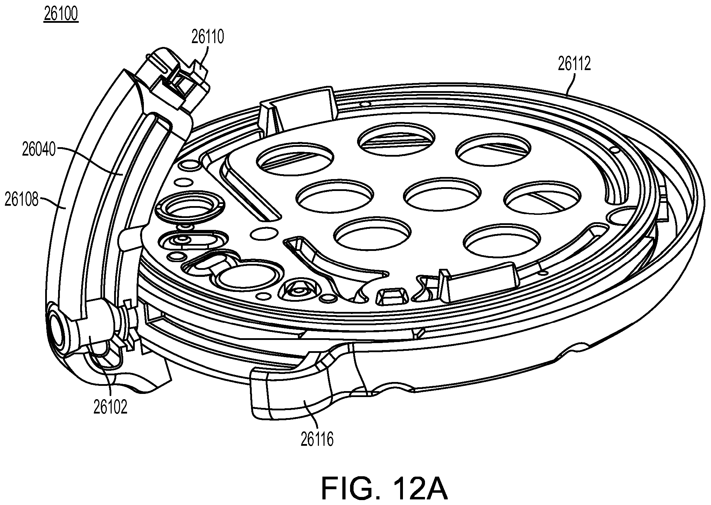

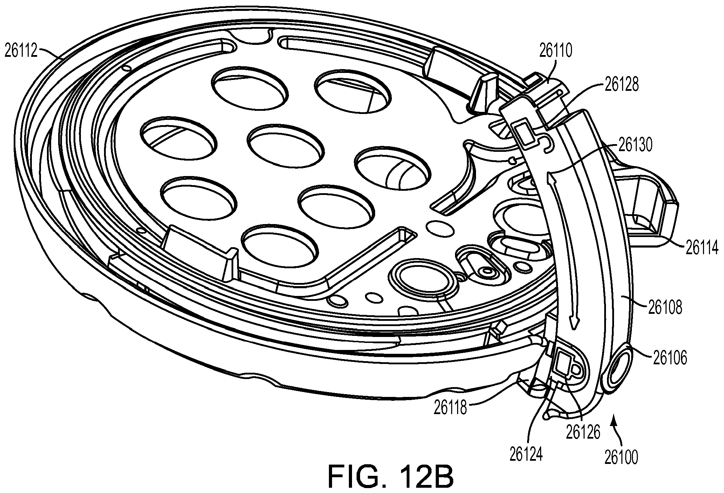

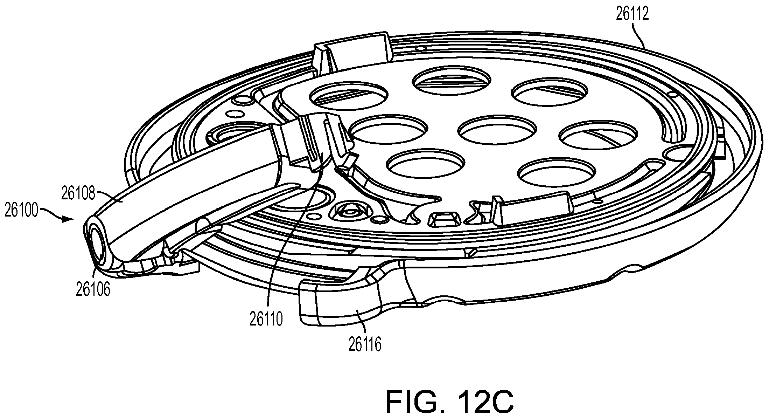

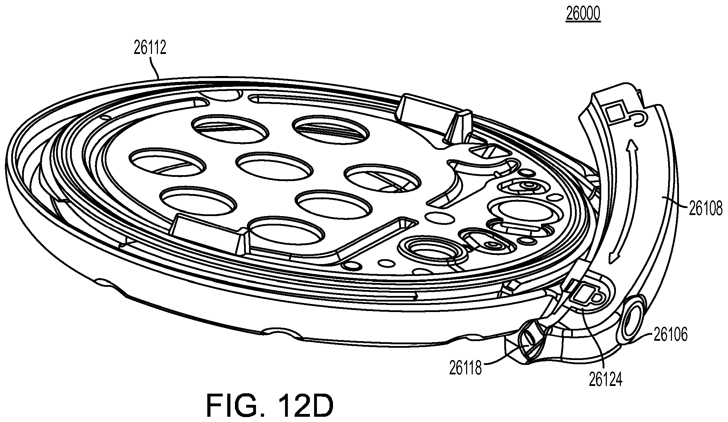

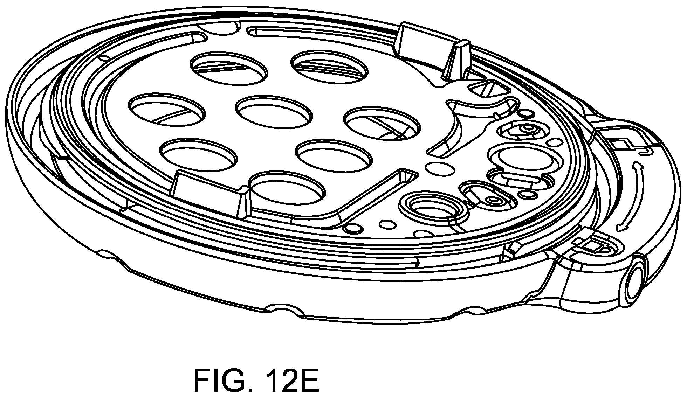

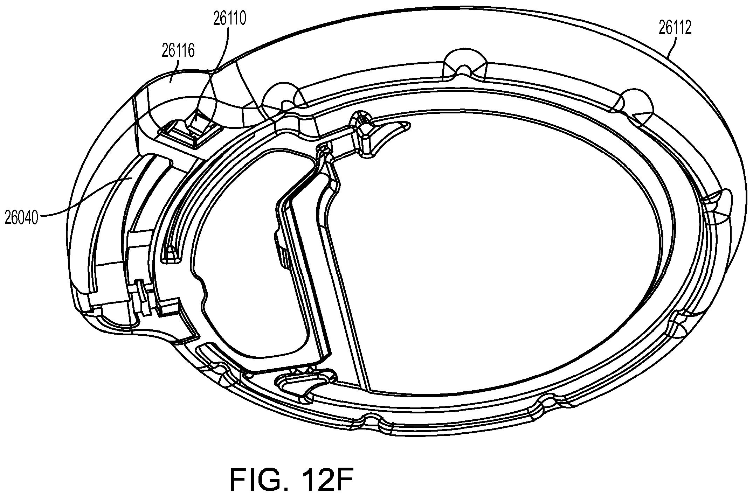

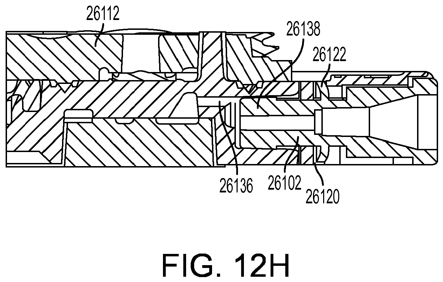

FIGS. 12A-12I are various views of a connector together with one embodiment of a disposable housing assembly according to an embodiment of the invention;

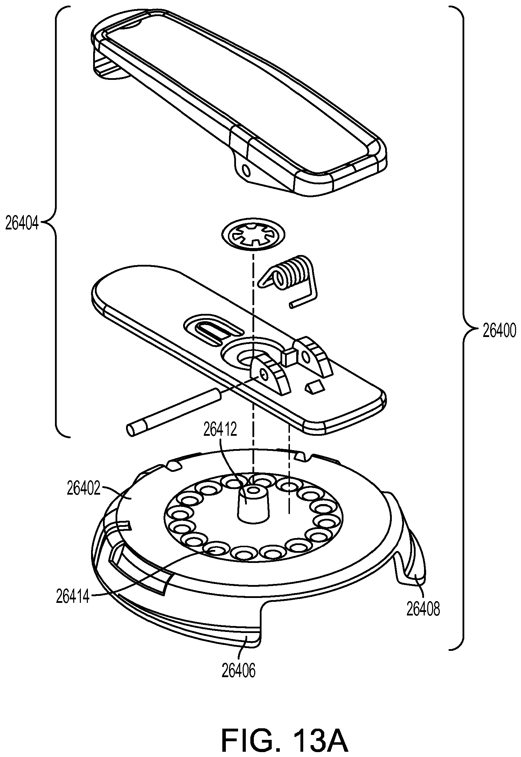

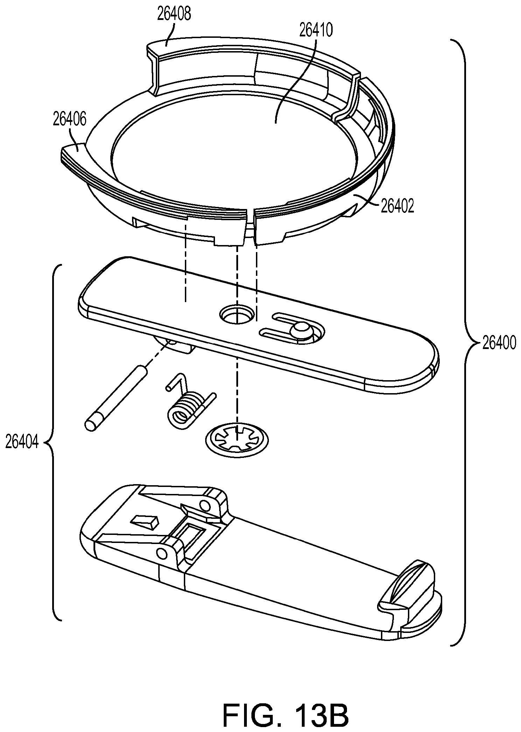

FIGS. 13A-13B are various views of an example of a clip assembly;

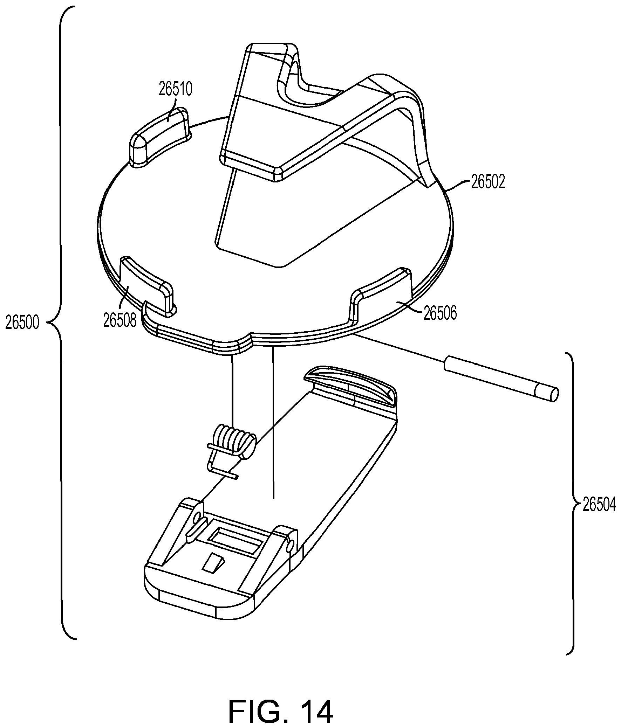

FIG. 14 is an exploded view of one example of a clip assembly;

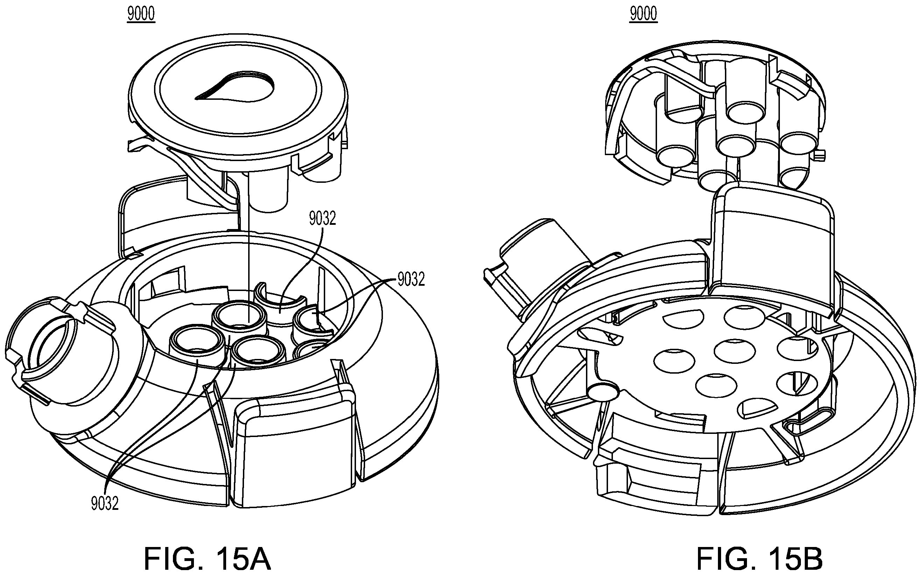

FIG. 15A is a top exploded view of one example of a fill adapter;
FIG. 15B is a bottom exploded view of one example of a fill adapter;

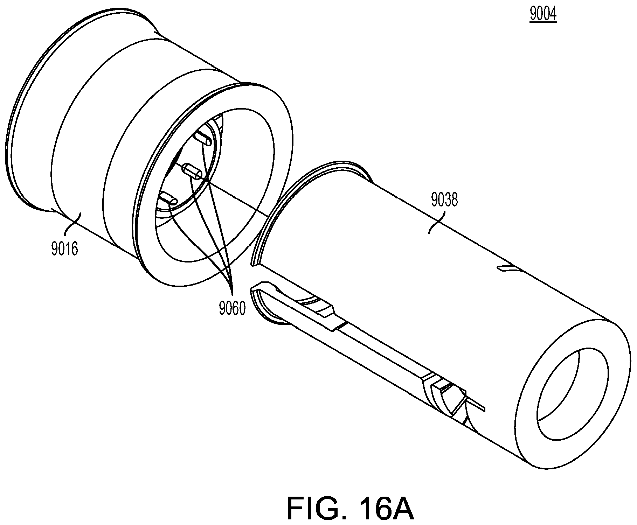

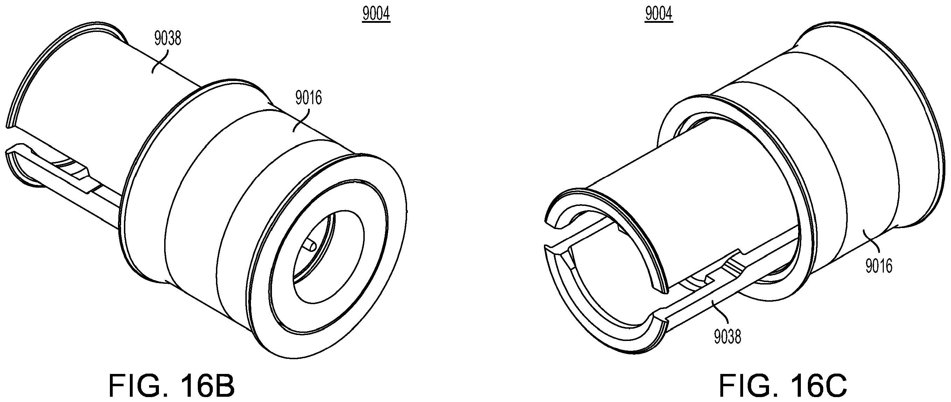

FIG. 16A is an exploded view of one example of a filling aid;
FIGS. 16B - 16C are isometric views of one example of a filling aid;

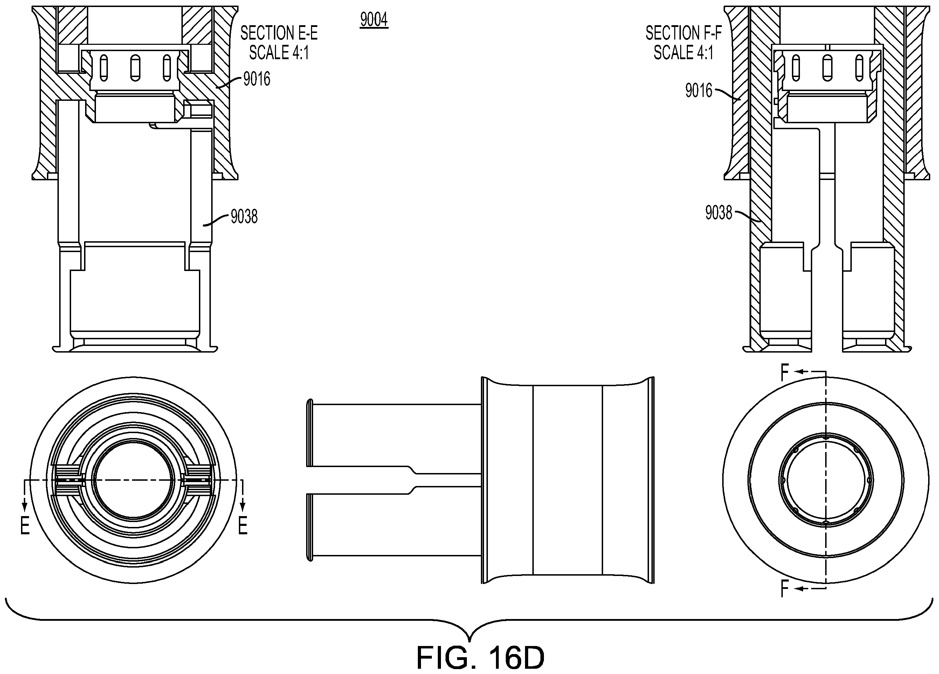

FIG. 16D are various views of one example of a filling aid;

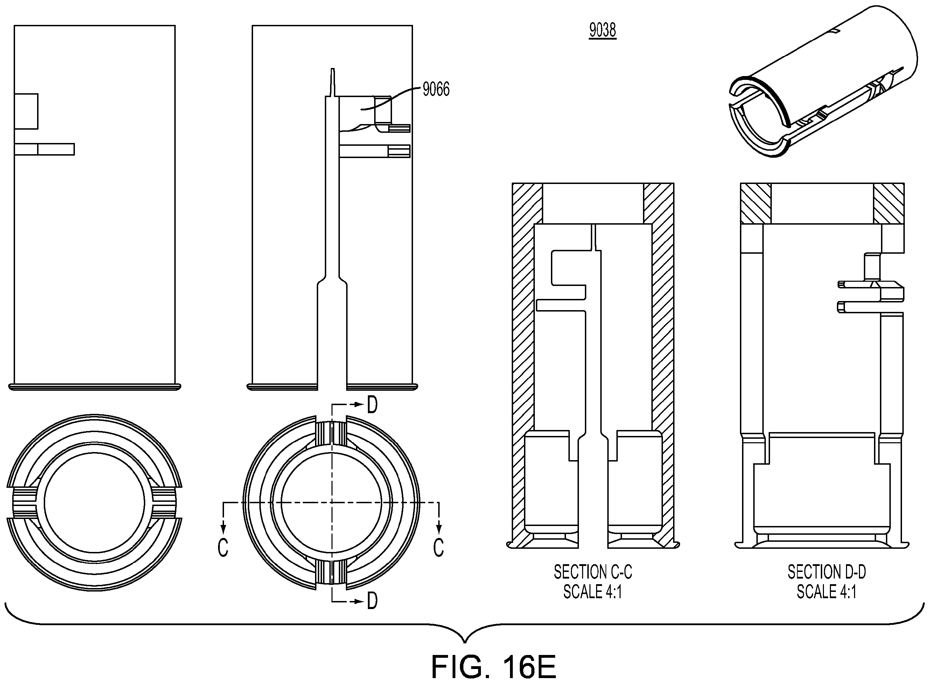

FIG. 16E are various views of one example of a needle housing;

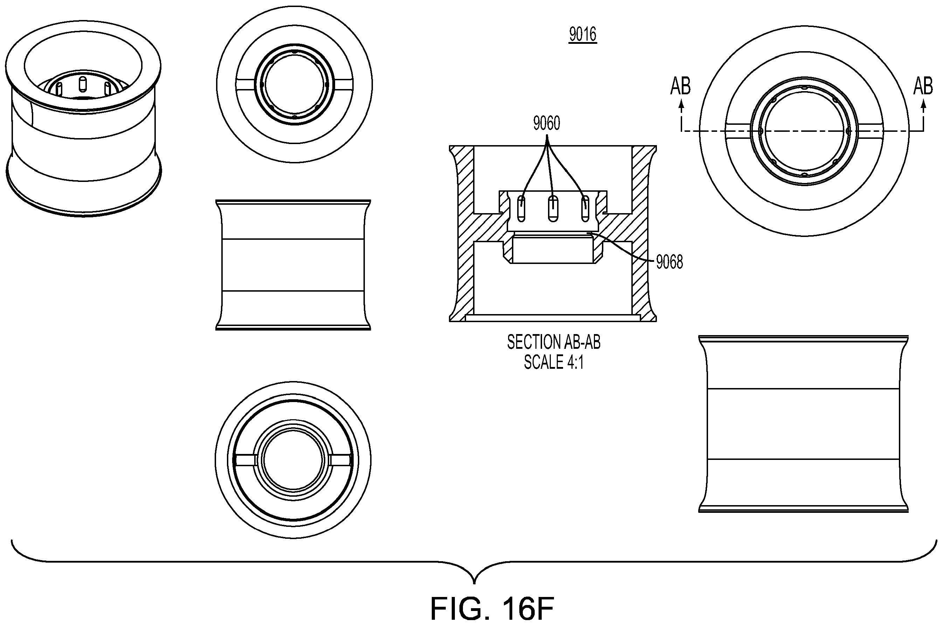

FIG. 16F are various views of one example of a filling needle cradle;

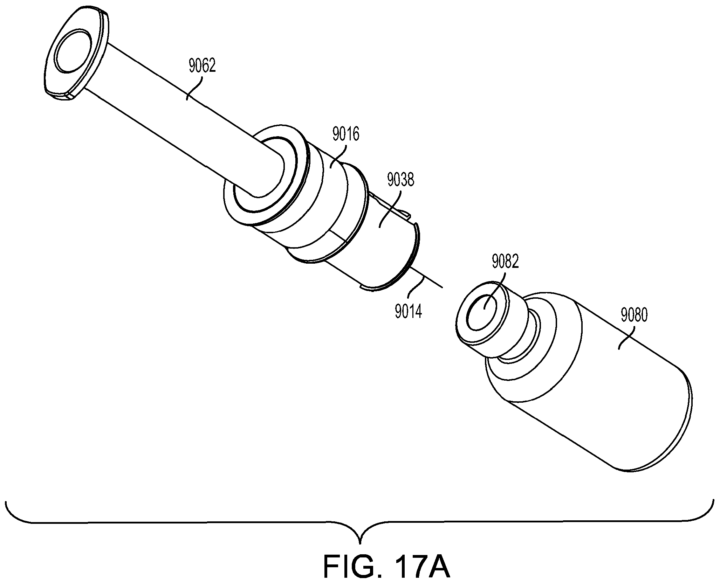

FIG. 17A is an isometric view of a filling syringe attached to a filling aid, also, a vial, according to one example;

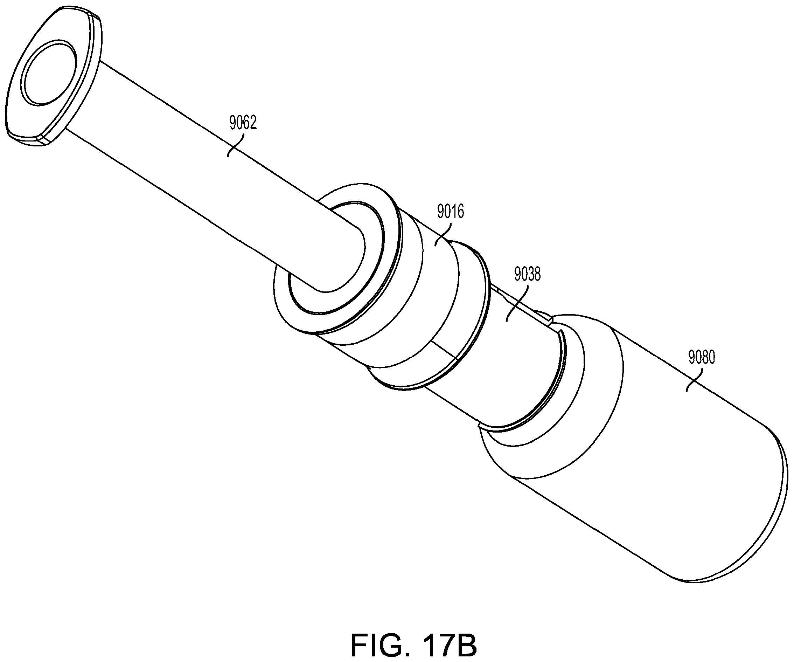

FIG. 17B is an isometric view of a filling syringe attached to both a filling aid and the filling aid attached to a vial, according to one example;

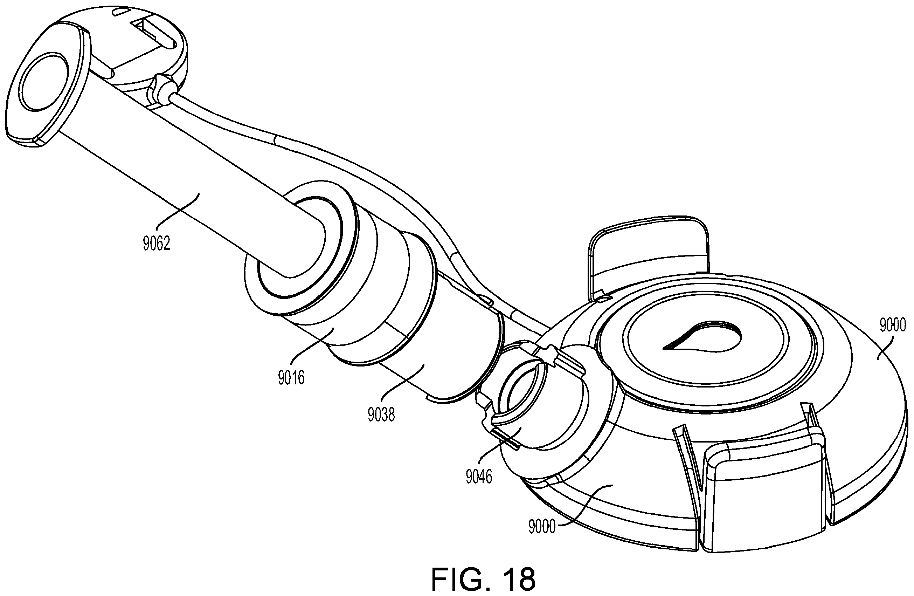

FIG. 18 is an isometric view of a fill adapter attached to a disposable housing assembly, and a filling syringe attached to a filling aid, according to one example;

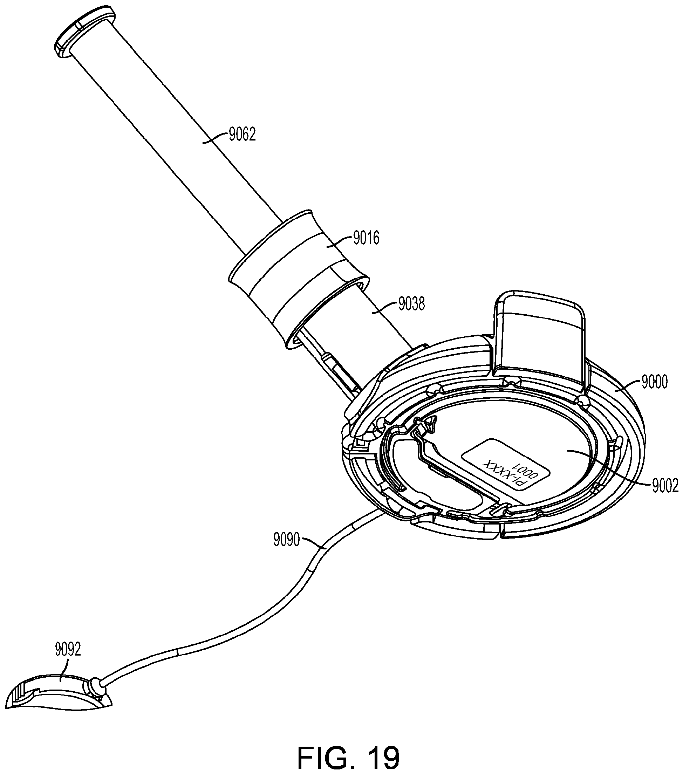

FIG. 19 is an isometric view of a fill adapter attached to a disposable housing assembly and to a filling aid, which is also attached to a filling syringe, according to one example;

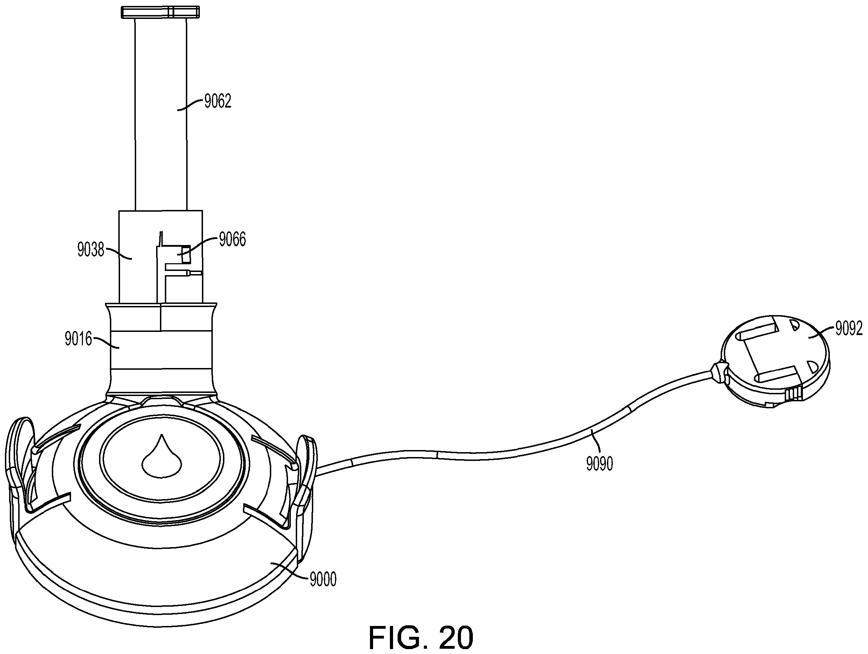

FIG. 20 is an isometric view of one example of a filling syringe, a filling aid and a fill adapter during the fill process of filling one example of a disposable housing assembly, according to one example;

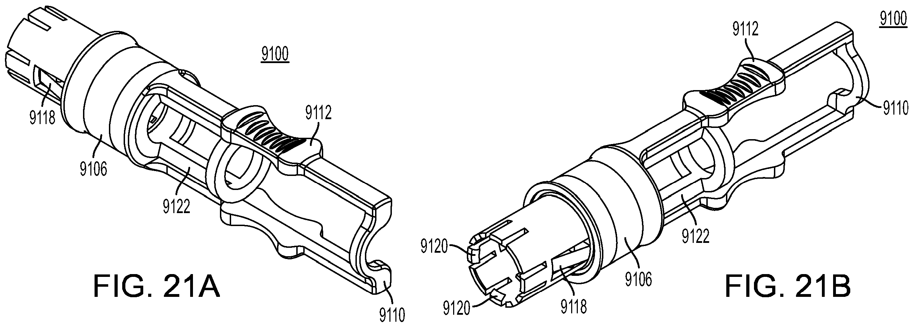

FIGS. 21A-21B are isometric views of a filling aid according to one example;

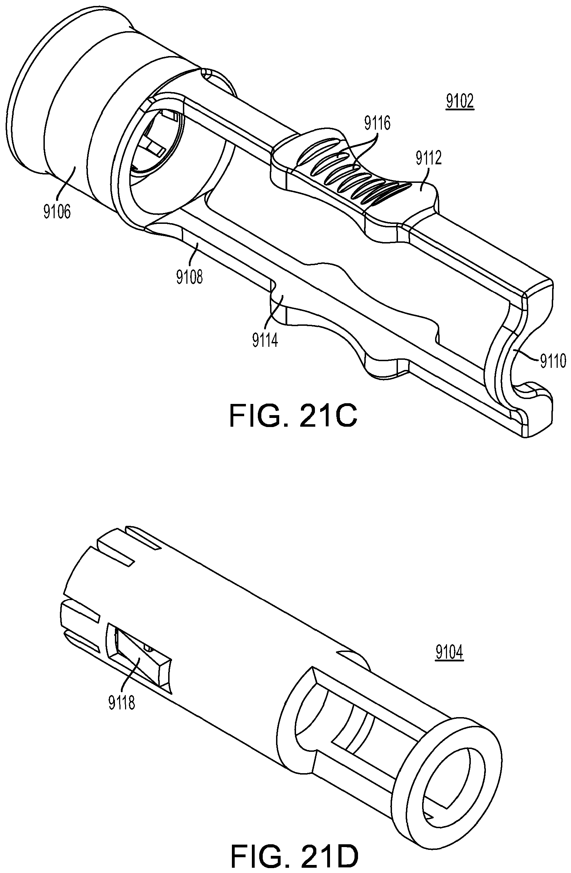

FIG. 21C is an isometric view of a filling syringe holder according to one example;
FIG. 21D is an isometric view of a locking portion according to one example;

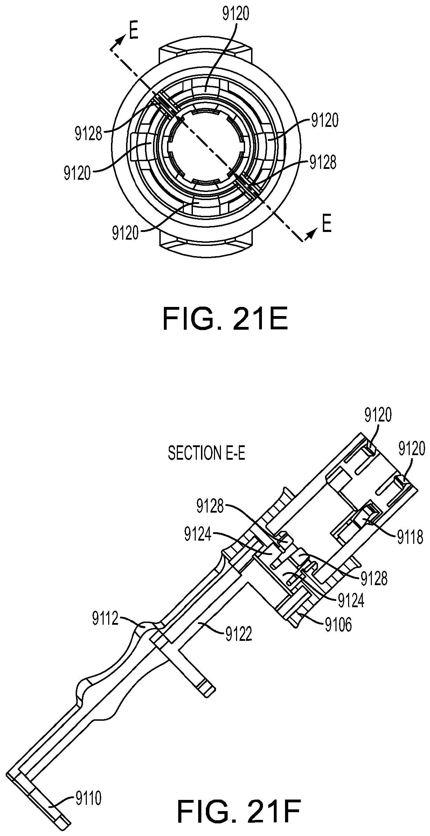

FIG. 21E is bottom view of a filling aid according to one example;
FIG. 21F is a cross sectional view of one example of a filling aid taken at section "E" shown in FIG. 21E;

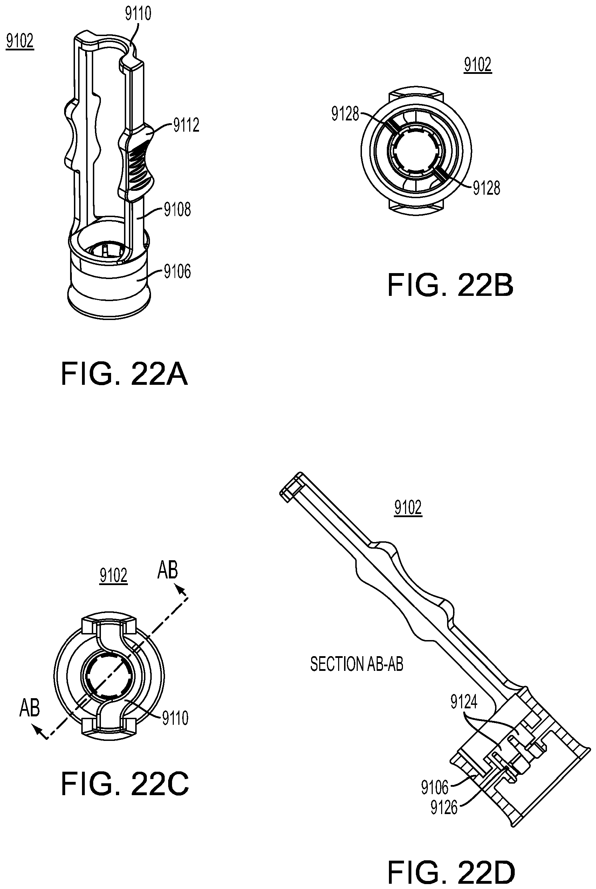

FIG. 22A is an isometric view of a filling syringe holder according to one example;
FIG. 22B is a bottom view of filling syringe holder according to one example;
FIG. 22C is a top view filling syringe holder according to one example;
FIG. 22D is a cross sections view of one example of a filling syringe holder taken at section "AB" shown in FIG. 22C;

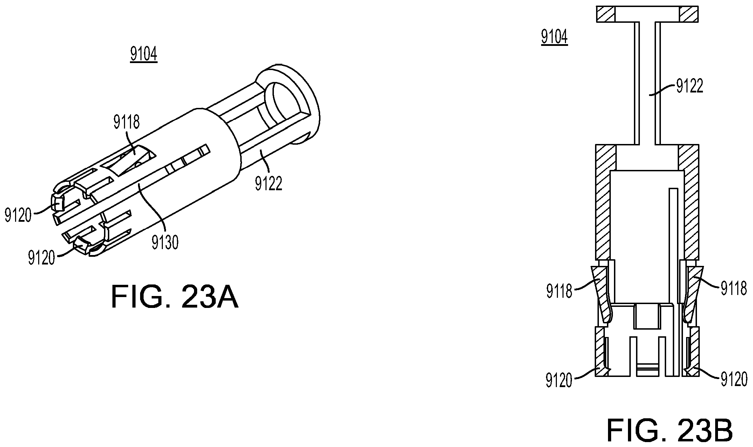

FIG. 23A is an isometric view of one example of a locking portion;
FIG. 23B is a cross sectional view of one example of a locking portion;

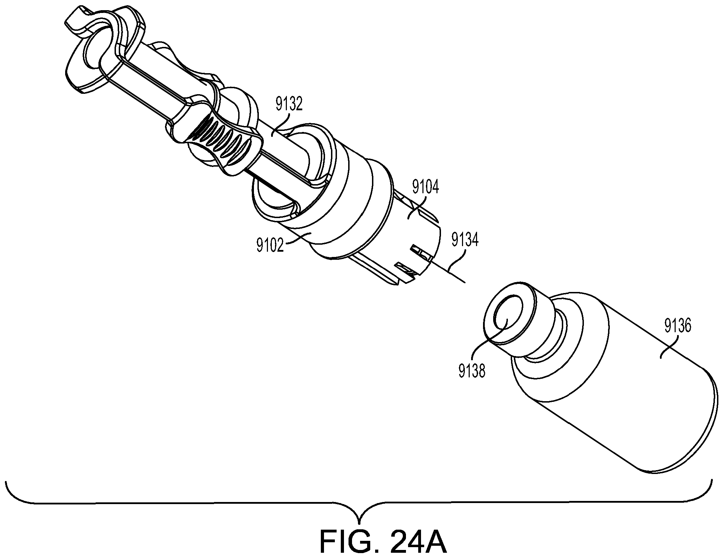

FIG. 24A is an isometric view of a filling syringe attached to a filling aid, also, a vial, according to one example;

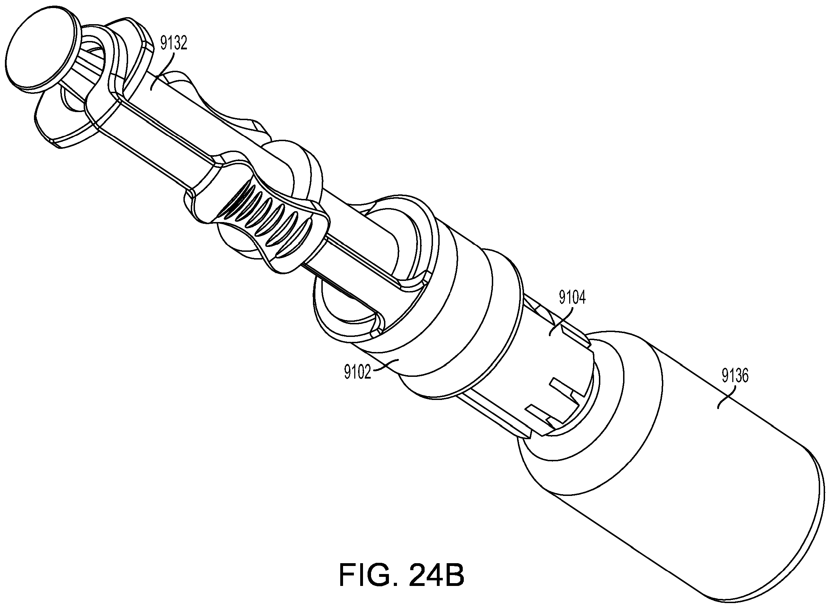

FIG. 24B is an isometric view of a filling syringe attached to both a filling aid and the filling aid attached to a vial, according to one example;

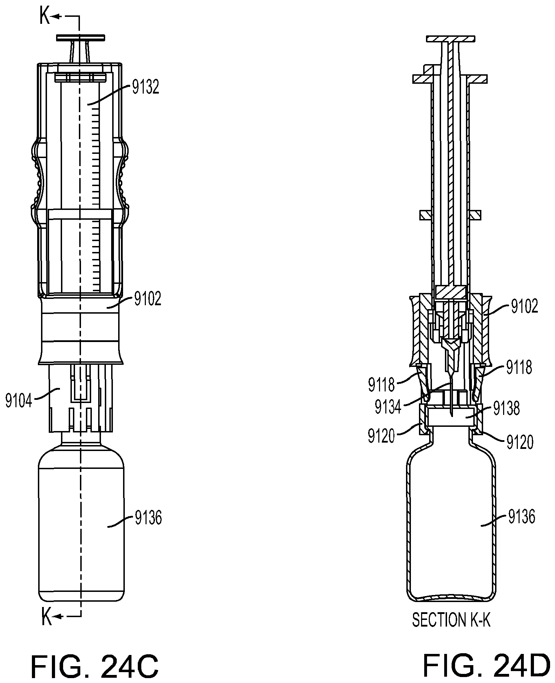

FIG. 24C is a view of a filling syringe attached to both a filling aid and the filling aid attached to a vial, according to one example;
FIG. 24D is a cross sectional view of one example of a filling syringe attached to both a filling aid and the filling aid attached to a vial;

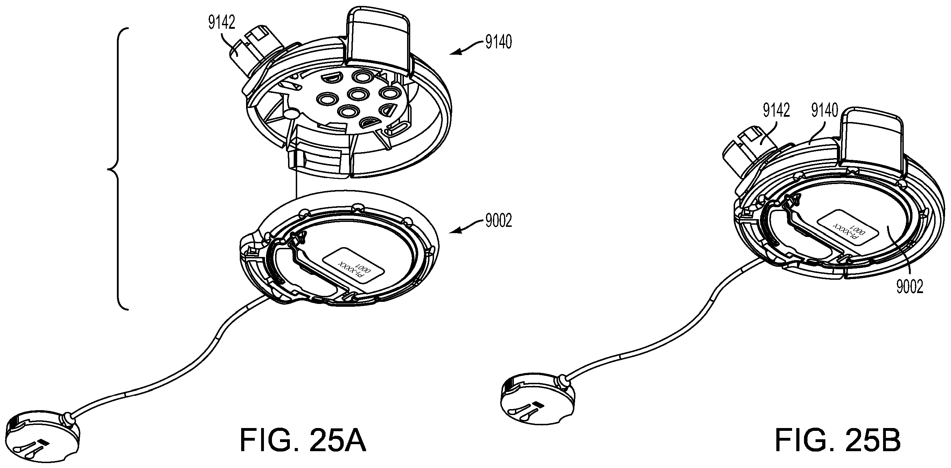

FIG. 25A is an exploded view of a fill adapter and a disposable housing assembly according to one example;
FIG. 25B is an isometric view of a fill adapter attached to a disposable housing assembly according to one example;

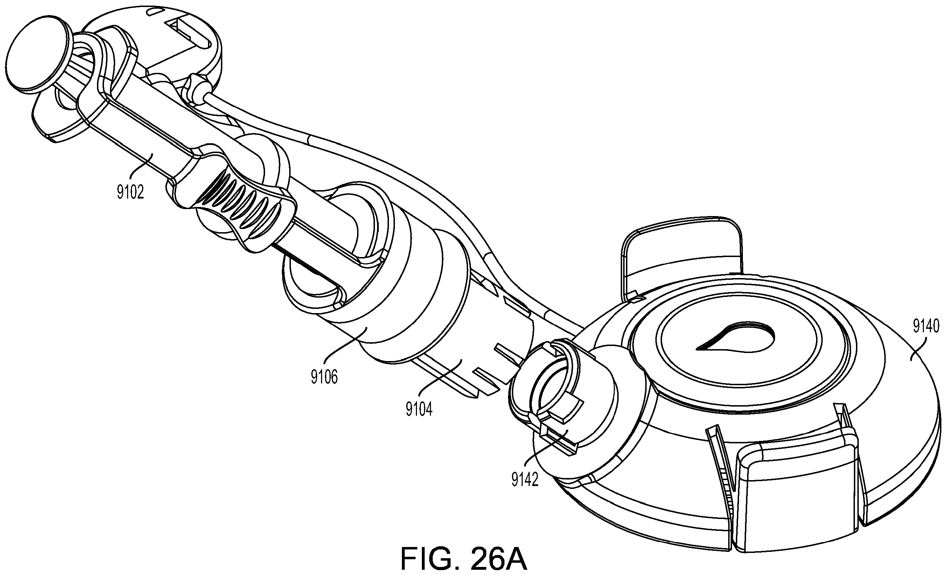

FIG. 26A is an isometric view of a fill adapter attached to a disposable housing assembly, and a filling syringe attached to a filling aid, according to one example;

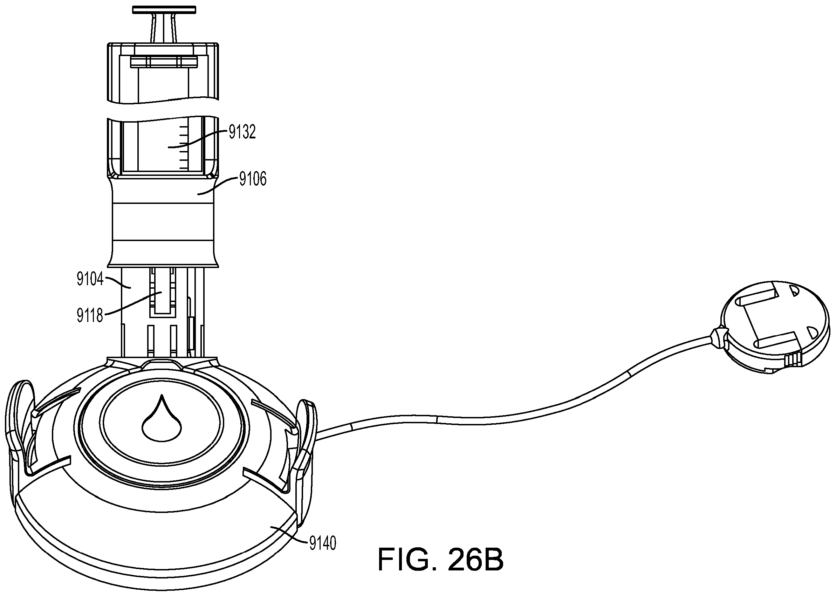

FIG. 26B is an isometric view of a fill adapter attached to a disposable housing assembly and to a filling aid, in the locked and starting position, which is also attached to a filling syringe, according to one example;

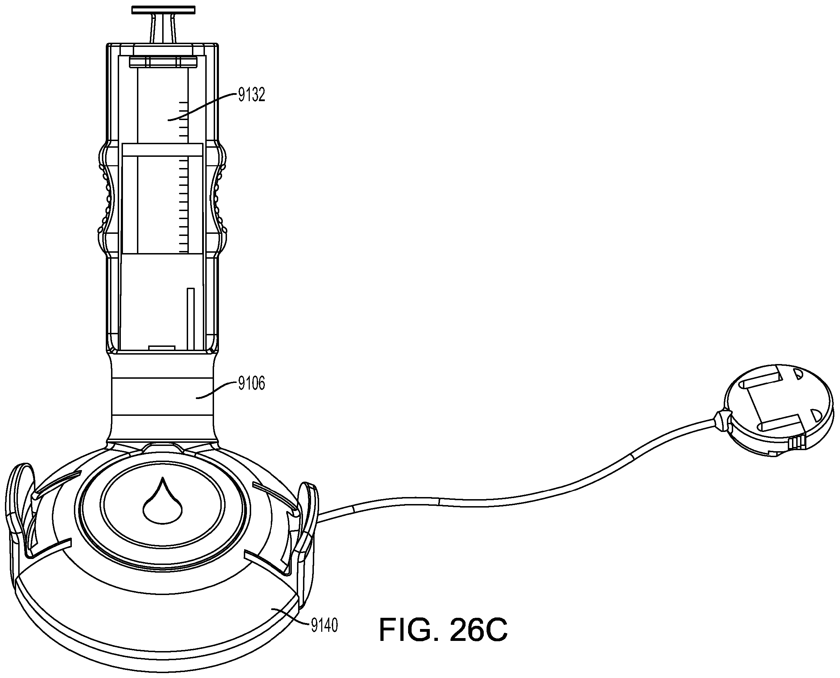

FIG. 26C is an isometric view of one example of a filling syringe, a filling aid in the unlocked and filling position and a fill adapter during the fill process of filling one example of a disposable housing assembly, according to one example;

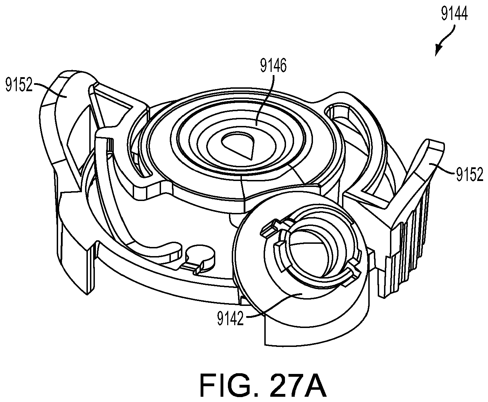

FIG. 27A is a to isometric view of one example of a fill adapter;

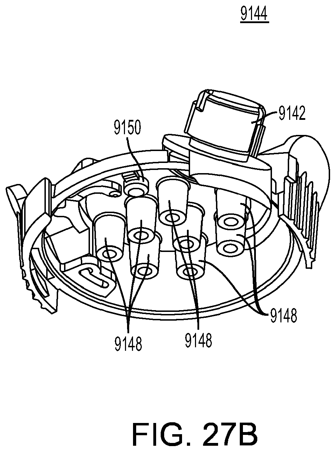

FIG. 27B is a bottom isometric view of one example of a fill adapter;

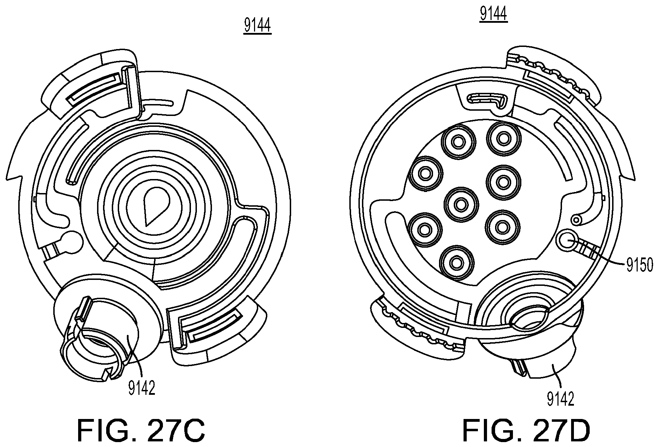

FIG. 27C is a top view of one example of a fill adapter;
FIG. 27D is a bottom view of one example of a fill adapter;

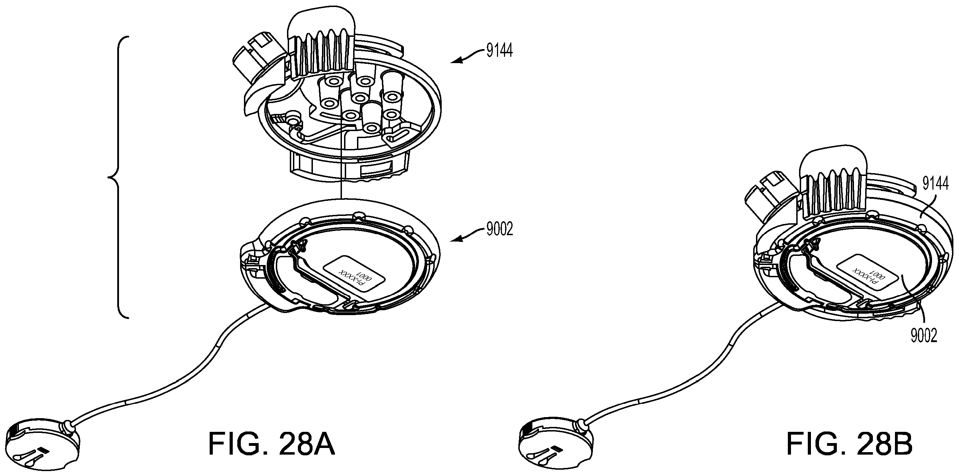

FIG. 28A is an exploded isometric view of a one example of a fill adapter and one example of a disposable housing assembly;
FIG. 28B is an isometric view of a one example of a fill adapter attached to one example of a disposable housing assembly; and

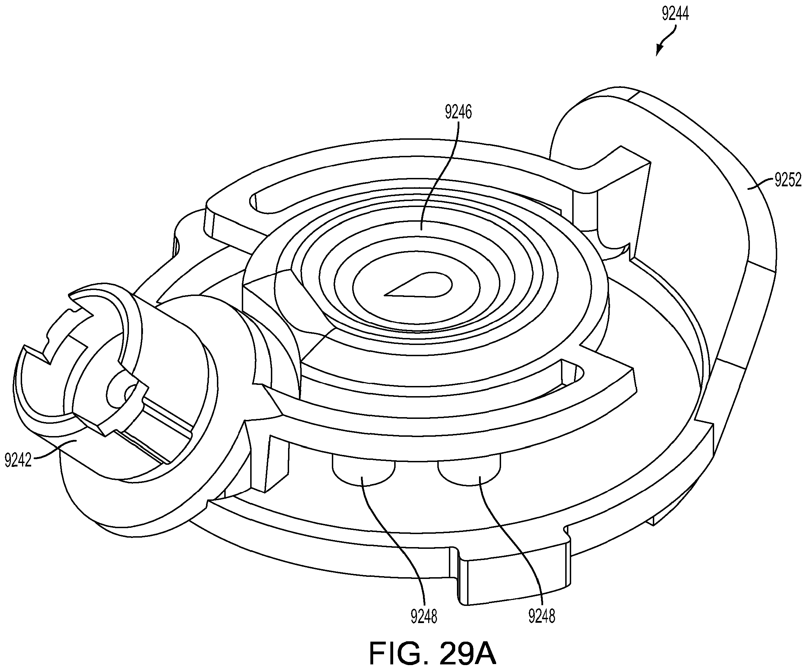

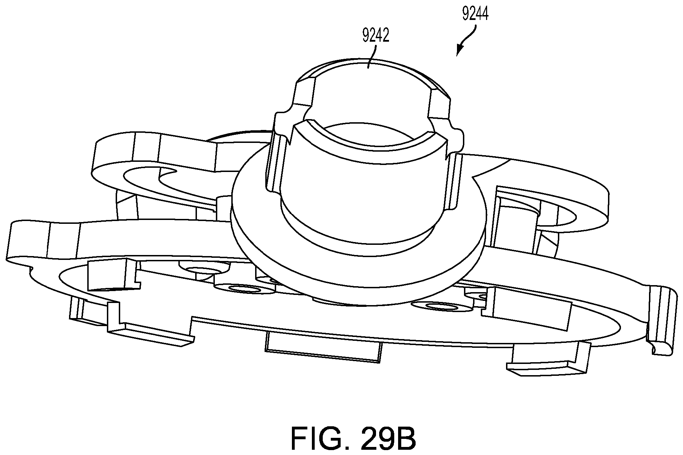

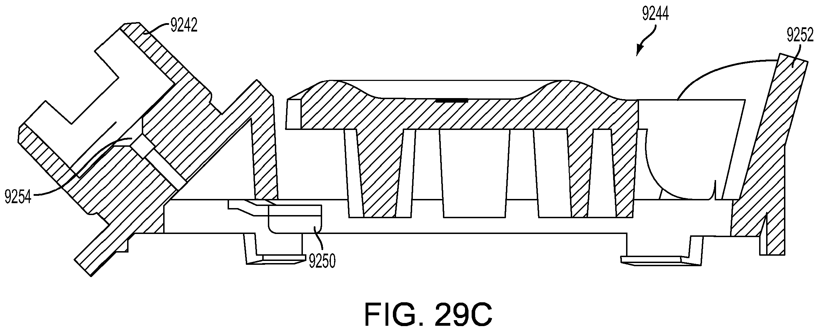

FIGS. 29A-29C show various views of one example of a fill adapter.

Like reference symbols in the various drawings indicate like elements.

### DETAILED DESCRIPTION

Referring to FIGS. 1-3, an infusion pump assembly 100 may include a reusable housing assembly 102. Reusable housing assembly 102 may be constructed from any suitable material, such as a hard or rigid plastic, that will resist compression. For example, use of durable materials and parts may improve quality and reduce costs by providing a reusable portion that lasts longer and is more durable, providing greater protection to components disposed therein.

Reusable housing assembly 102 may include mechanical control assembly 104 having a pump assembly 106 and at least one valve assembly 108. Reusable housing assembly 102 may also include electrical control assembly 110 configured to provide one or more control signals to mechanical control assembly 104 and effectuate the basal and/ or bolus delivery of an infusible fluid to a user. Disposable housing assembly 114 may include valve assembly 108 which may be configured to control the flow of the infusible fluid through a fluid path. Reusable housing assembly 102 may also include pump assembly 106 which may be configured to pump the infusible fluid from the fluid path to the user.

Electrical control assembly 110 may monitor and control the amount of infusible fluid that has been and/or is being pumped. For example, electrical control assembly 110 may receive signals from volume sensor assembly 148 and calculate the amount of infusible fluid that has just been dispensed and determine, based upon the dosage required by the user, whether enough infusible fluid has been dispensed. If enough infusible fluid has not been dispensed, electrical control assembly 110 may determine that more infusible fluid should be pumped. Electrical control assembly 110 may provide the appropriate signal to mechanical control assembly 104 so that any additional necessary dosage may be pumped or electrical control assembly 110 may provide the appropriate signal to mechanical control assembly 104 so that the additional dosage may be dispensed with the next dosage. Alternatively, if too much infusible fluid has been dispensed, electrical control assembly 110 may provide the appropriate signal to mechanical control assembly 104 so that less infusible fluid may be dispensed in the next dosage.

Mechanical control assembly 104 may include at least one shape-memory actuator 112. Pump assembly 106 and/or valve assembly 108 of mechanical control assembly 104 may be actuated by at least one shape-memory actuator, e.g., shape-memory actuator 112, which may be a shape-memory wire in wire or spring configuration. Shape memory actuator 112 may be operably connected to and activated by electrical control assembly 110, which may control the timing and the amount of heat and/or electrical energy used to actuate mechanical control assembly 104. Shape memory actuator 112 may be, for example, a conductive shape-memory alloy wire that changes shape with temperature. The temperature of shape-memory actuator 112 may be changed with a heater, or more conveniently, by application of electrical energy. Shape memory actuator 112 may be a shape memory wire constructed of nickel/titanium alloy, such as NITINOL^{™} or FLEXINOL^{®}.

Infusion pump assembly 100 may include a volume sensor assembly 148 configured to monitor the amount of fluid infused by infusion pump assembly 100. For example, volume sensor assembly 148 may employ, for example, acoustic volume sensing. Acoustic volume measurement technology is the subject of U.S. Patent Nos. 5,575,310 and 5,755,683 assigned to DEKA Products Limited Partnership, as well as U.S. patent application Publication Nos. US 2007/0228071 A1, US 2007/0219496 A1, US 2007/0219480 A1, US 2007/0219597 A1. Other alternative techniques for measuring fluid flow may also be used; for example, Doppler-based methods; the use of Hall-effect sensors in combination with a vane or flapper valve; the use of a strain beam (for example, related to a flexible member over a fluid reservoir to sense deflection of the flexible member); the use of capacitive sensing with plates; or thermal time of flight methods. One such alternative technique is disclosed in U.S. Patent Application Serial No. 11/704,899 filed February 9, 2007, now U.S. Publication No. US-2007-0228071-A1 published October 4, 2007 and entitled Fluid Delivery Systems and Methods (Attorney Docket No. E70). Infusion pump assembly 100 may be configured so that the volume measurements produced by volume sensor assembly 148 may be used to control, through a feedback loop, the amount of infusible fluid that is infused into the user.

Infusion pump assembly 100 may further include a disposable housing assembly 114. For example, disposable housing assembly 114 may be configured for a single use or for use for a specified period of time, e.g., three days or any other amount of time. Disposable housing assembly 114 may be configured such that any components in infusion pump assembly 100 that come in contact with the infusible fluid are disposed on and/or within disposable housing assembly 114. For example, a fluid path or channel including a reservoir, may be positioned within disposable housing assembly 114 and may be configured for a single use or for a specified number of uses before disposal. The disposable nature of disposable housing assembly 114 may improve sanitation of infusion pump assembly 100.

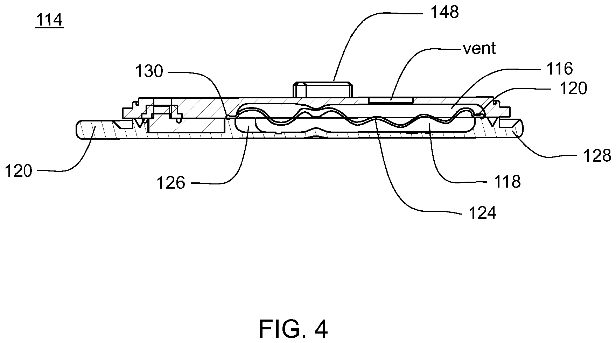

Referring also to FIG. 4, disposable housing assembly 114 may be configured to releasably engage reusable housing assembly 102, and includes a cavity 116 that has a reservoir 118 for receiving an infusible fluid (not shown), e.g., insulin. Such releasable engagement may be accomplished by a screw-on, a twist-lock or a compression fit configuration, for example. Disposable housing assembly 114 and/or reusable housing assembly 102 may include an alignment assembly configured to assist in aligning disposable housing assembly 114 and reusable housing assembly 102 for engagement in a specific orientation. Similarly, base nub 120 and top nub 122 may be used as indicators of alignment and complete engagement.

Cavity 116 may be at least partially formed by and integral to disposable housing assembly 114. Cavity 116 may include a membrane assembly 124 for at least partially defining reservoir 118. Reservoir 118 may be further defined by disposable housing assembly 114, e.g., by a recess 126 formed in base portion 128 of disposable housing assembly 114. For example, membrane assembly 124 may be disposed over recess 126 and attached to base portion 128, thereby forming reservoir 118. Membrane assembly 124 may be attached to base portion 128 by conventional means, such as gluing, heat sealing, and/or compression fitting, such that a seal 130 is formed between membrane assembly 124 and base portion 128. Membrane assembly 124 may be flexible and the space formed between membrane assembly 124 and recess 126 in base portion 128 may define reservoir 118. Reservoir 118 may be non-pressurized and in fluid communication with a fluid path (not shown). Membrane assembly 124 may be at least partially collapsible and cavity 116 may include a vent assembly, thereby advantageously preventing the buildup of a vacuum in reservoir 118 as the infusible fluid is delivered from reservoir 118 to the fluid path. In a preferred embodiment, membrane assembly 124 is fully collapsible, thus allowing for the complete delivery of the infusible fluid. Cavity 116 may be configured to provide sufficient space to ensure there is always some air space even when reservoir 118 is filled with infusible fluid.

The membranes and reservoirs described herein may be made from materials including but not limited to silicone, NITRILE, butyl rubber, SANTOPRENE, thermal plastic elastomers (TPE), styrene ethylene butylene styrene (SEBS) and / or any other material having desired resilience and properties for functioning as described herein. Additionally, other structures could serve the same purpose.

The use of a partially collapsible non pressurized reservoir may advantageously prevent the buildup of air in the reservoir as the fluid in the reservoir is depleted. Air buildup in a vented reservoir could prevent fluid egress from the reservoir, especially if the system is tilted so that an air pocket intervenes between the fluid contained in the reservoir and the septum of the reservoir. Tilting of the system is expected during normal operation as a wearable device.

Reservoir 118 may be conveniently sized to hold an insulin supply sufficient for delivery over one or more days. For example, reservoir 118 may hold about 1.00 to 3.00 ml of insulin. A 3.00 ml insulin reservoir may correspond to approximately a three day supply for about 90% of potential users. In other embodiments, reservoir 118 may be any size or shape and may be adapted to hold any amount of insulin or other infusible fluid. In some embodiments, the size and shape of cavity 116 and reservoir 118 is related to the type of infusible fluid that cavity 116 and reservoir 118 are adapted to hold.

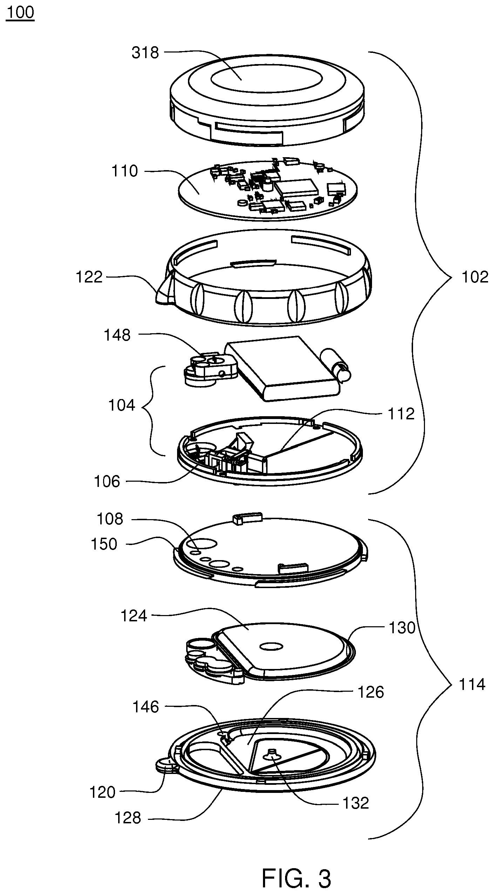

Disposable housing assembly 114 may include a support member 132 (FIG. 3) configured to prevent accidental compression of reservoir 118. Compression of reservoir 118 may result in an unintentional dosage of infusible fluid being forced through the fluid path to the user. In a preferred embodiment, reusable housing assembly 102 and disposable housing assembly 114 may be constructed of a rigid material that is not easily compressible. However, as an added precaution, support member 132 may be included within disposable housing assembly 114 to prevent compression of infusion pump assembly 100 and cavity 116 therein. Support member 132 may be a rigid projection from base portion 128. For example, support member 132 may be disposed within cavity 116 and may prevent compression of reservoir 118.

As discussed above, cavity 116 may be configured to provide sufficient space to ensure there is always some air space even when reservoir 118 is filled with infusible fluid. Accordingly, in the event that infusion pump assembly 100 is accidentally compressed, the infusible fluid may not be forced through cannula assembly 136.

Cavity 116 may include a septum assembly 146 (FIG. 3) configured to allow reservoir 118 to be filled with the infusible fluid. Septum assembly 146 may be a conventional septum made from rubber or plastic and have a one-way fluid valve configured to allow a user to fill reservoir 118 from a syringe or other filling device. In some embodiments, septum 146 may be located on the top of membrane assembly 124. In these embodiments, cavity 116 may include a support structure (e.g., support member 132 in FIG. 3) for supporting the area about the back side of the septum so as to maintain the integrity of the septum seal when a needle is introducing infusible fluid into cavity 116. The support structure may be configured to support the septum while still allowing the introduction of the needle for introducing infusible fluid into cavity 116.

Various examples of the connector (or fluid connector, said terms may be used interchangeably) may include a plug (or post, namely which may be used interchangeably throughout this specification) that may include one or more features. In various embodiments, the plug may connect to the disposable housing assembly through a luer connection. Referring now to FIGS. 5A-5C, in various embodiments, any one or more embodiments of the plug 25600 may include (either in addition to one or more features described above or instead of one or more features described above) one more tab features 25602, 25604. In some embodiments, for example, as shown in FIGS. 5C-5C, various embodiments of the plug 25600 may include two tab features 25606, 25608 on either side of the plug 25600. In various embodiments, the tab features 24602, 25604, 25606, 25608 may take up "play" or "wiggle" between the plug 25600 and the disposable housing assembly exit. In various embodiments, and referring also to FIG. 5D, the disposable housing assembly 25610 may include slots 25612, 25614 at the exit 25616 to accommodate the tab features 24602, 25604, 25606, 25608. In some embodiments, the slots 25612, 25614 may be ramped, which may be desirable/beneficial for many reasons, including, but not limited to, the ramped slots 25612, 25614 may assist in the plug 25600 moving further into the disposable housing assembly 25610. Once inside the disposable housing assembly 25610, the tabs with bend such that the plug 25600 and the exit 25616 form a luer connection. In some embodiments the tab features 24602, 25604, 25606, 25608 therefore may drive the plug 25600 as far into the exit 25616 as it may go. In some embodiments, the tab features 24602, 25604, 25606, 25608 are angled with respect to the plug 25600 such that the angle is smaller than 180 degrees, upon entry into the exit 25616. As the plug 25600 enters further into the exit 25616, the tab features 24602, 25604, 25606, 25608 move such that they are flattened against the plug 25600. This may be desirable/beneficial for many reasons, including, but not limited to, the plug 25600 may only rotate with respect to the disposable housing assembly 25610 when the tab features 24602, 25604, 25606, 25608 are flattened, thus ensuring that the plug 25600 is all the way inside the exit 25616 of the disposable housing assembly 25610 before the connector is locked to the disposable housing assembly 25610. This may be desirable/beneficial for many reasons, including, but not limited to, preventing leaks. In various embodiments of this embodiment of the plug 25600, the plug 25600 may be rigid, i.e. not include an elastomer, and the tab features 24602, 25604, 25606, 25608 may be made from a rigid material that is thin enough to bend as described above. However, in various other embodiments, the plug 25600 may be made from any one or more of the various materials described herein with respect to the various embodiments of the plug. In some embodiments, the plug 25600 may be made from TERLUX^{®} and/or another ABS material.

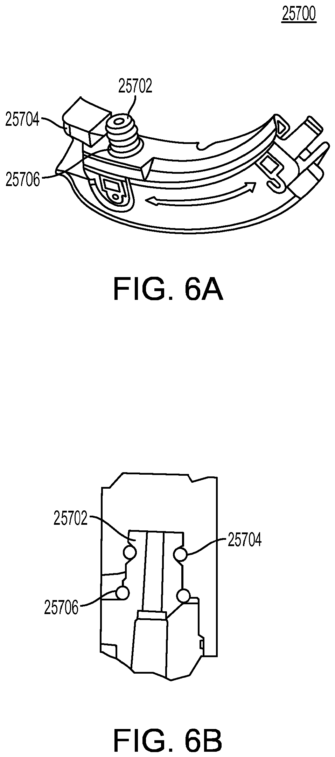

Referring now also to FIGS. 6A-6B, in various embodiments, the connector 25700 includes a plug 25700 that may include one or more seals. In some embodiments, as discussed above, the seals may be any type of seal, including, but not limited to, a face seal and or a radial seal. In some embodiments, such as those shown in FIGS. 6A-6B, the plug 25700 may include a radial seal 25702 and a face seal 25706. Thus, in some embodiments, the plug 25700 may include two seals, one having a larger diameter than the other.

Referring now also to FIGS. 7A - 7B, in various embodiments, the connector 25800 may include a plug 25800 that includes a single elastomer seal 25804 including a radial seal portion 25806 and a face seal portion 25810 separated by an elastomer portion. These embodiment may be beneficial/desirable for many reasons, including, but not limited to, this embodiment allows for the removal of all parting lines on the plug 25802 and the elastomer seal 25804. This may be beneficial/desirable for many reasons, including, but not limited to, removing the leak paths that may be present due to parting lines.

Referring now to FIGS. 8A-8C, in various embodiments, the connector 25900 may include a plug 25902 that includes a seal 25904 that may be an elastomer and includes two rings 25906, 25908 separated by an elastomer section. Various embodiments of the plug may include a double seal. The plug 25902 is shown without the seal in FIG. 8C. In this embodiment of the plug 25902, there are no parting lines on the surface of the plug 2590. This may be beneficial/desirable for many reasons, including, but not limited to, removing the leak paths that may be present due to parting lines.

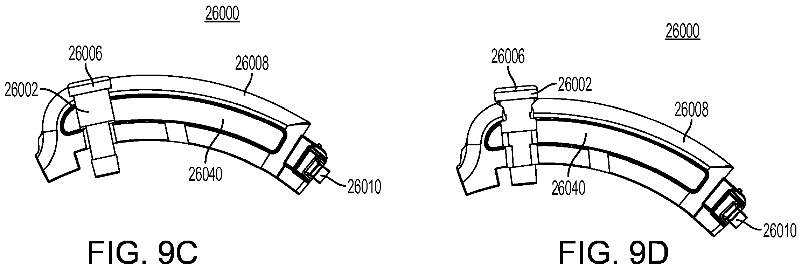

Referring now also to FIGS. 9A - 9G, some embodiments of the connector 26000 include a plug 26002 that is rigid and forms a luer connection with the exit of the disposable housing assembly. In various embodiments, the plug 26002 may be tapered, and the percentage of tapering may vary. In some embodiments, the taper may be 6%, however, in various other embodiments, the taper may be greater or less than 6%. In embodiments, including the one shown in FIGS. 9A - 9G, the plug 26002 is removably attached to the connector 26000 and, in addition, is slidably connected to the connector 26000. Thus, as shown in FIGS. 9B and 9C, the plug 26002 may slide from an initial position, shown in FIG. 9B, to a final position, shown in FIG. 9D with respect to the tab portion of the connector 26008. In various embodiments, the tab portion has a first side or reservoir side and a second side or tubing side and the plug may slide in the direction of the tubing side. In embodiments, the plug 26002 is configured such that it includes at least one stop feature wherein the exit end portion 26004 of the plug 26002 includes a first stop feature, for example, a lip, that limits the travel of the plug 26004 in one direction. The plug 26002 tubing end portion 26006 in various embodiments also includes a second stop feature, for example, a lip, that limits the travel of the plug 26002 in the other direction. In various embodiments, the plug 26002 may be made from any material, for example, but not limited to, TERLUX^{®} and/or another ABS material. Although not shown in the embodiments shown in FIGS. 9A-9D, the plug 26002 includes a length of tubing attached to the tubing end portion 26006, which tubing and attachment may be any one or more of the various embodiments described in more detail above with respect to various embodiments of the plug and the connector. The length of tubing may be any length desired, including, but not limited to, any length between 1 inch and 10 feet, and in some embodiments may be either 4 inches or 23 inches, however, in various embodiments, the length may be greater or less than 4 inches or 23 inches.

In various embodiments, the connector 26000 includes a tab portion 26008 including a mating locking feature 26010, which may be, in various embodiments, the mating locking feature described above with respect to various other embodiments of the connector 26000. In various embodiments, the mating locking feature 26010 is configured to mate with a corresponding feature on the disposable housing assembly 26012.

In various embodiments, the plug 26002 of the connector 26000 is inserted into the disposable housing assembly 26012 with the tab portion 26008 (or body portion, which may be used interchangeably with the term tab portion) pointing upward. Once the plug 26002 portion is inside the disposable housing assembly 26012, the tab portion 26008 may be rotated about the plug 26002 to rest above the disposable housing assembly 26012 adjacent to the exit (i.e., adjacent to the tab portion 26016 on the disposable housing assembly). In some embodiments, mating locking features 26010, 26014 may be included on the tab portion 26008 of the connector 26000 and the disposable housing assembly 26012 such that the connector 26000 is held in place before the reusable housing assembly (not shown, shown and described as 6004 above) is attached to the disposable housing assembly 26012. In some embodiments, the mating locking features 26010, 26014 may include, but are not limited to, snap buttons and / or catch features. In some embodiments, a hook or other feature may be located on the opposite end of the tab 26008 portion of the connector 26000 such that it loops over the end of the tab portion 26016 of the disposable housing assembly 26012 and maintains the position of the connector 26000.

Still referring also to FIGS. 9A-9G, another connector 26000 is shown. The connector 26000 may include a tab portion 26008, a catch feature 26018, a latching feature 26010, and a plug 26002. In some embodiments, the tab portion 26008 of the connector 26000 may include mating locking features that interact with corresponding features in the disposable housing assembly 26012. In some embodiments, the tab portion 26008 may include features that interact with other portions of the disposable housing assembly 26012. As shown, in some embodiments, the connector 26000 may include a male latching feature 26010 on the end of the tab portion 26008 of the connector 26000. In some embodiments, the latching feature 26010 may mate with a corresponding latching feature (a female latching feature 26014) on the disposable housing assembly 26012. In some embodiments, the latch may be a removably secure fit and / or a snap fit and/or a loose snap fit and in some embodiments may include a spring latch portion. In various other embodiments, the shape and size of the connector 26000 may vary and/or in various other embodiments, other types of features such as mating locking features may be used, which include, but are not limited to, latches, catches, snap fits, adhesives, and other mechanisms for securing a connector 26000 to the tab 26016 of the disposable housing assembly 26012.

In some embodiments, the connector 26000 may include a locking ring feature on the underside. The locking ring feature in some embodiments may be tapered and/or in some embodiments, the locking ring feature may be at least slightly curved. In some embodiments, the locking ring feature may interact with the locking ring of the reusable housing assembly and may act together with the locking ring to secure the connector to the disposable housing assembly.

As discussed above, in various embodiments, the plug 26002 of the connector 26000 is inserted into the exit of the disposable housing assembly 26012 with the tab portion 26008 pointing in the general upward direction. Once the plug 26002 is inside the exit, the tab portion 26008 of the connector 26000 may be rotated to rest adjacent to the tab portion 26016 of the disposable housing assembly 26012. In some embodiments, mating locking features may be included on the tab portion 26008 of the connector 26000 and the disposable housing assembly 26012 such that the connector 26000 is held in place before the reusable housing assembly is attached to the disposable housing assembly 26012. In some embodiments, the mating locking features may include, but are not limited to, post and opening, snap, buttons, latch features and / or catch features. In some embodiments, a hook or other feature may be located on the opposite end of the tab portion 26008 of the connector 26000 such that it loops over the end of the tab portion 26016 of the disposable housing assembly 26012 and maintains the position of the connector 26000. In the embodiment shown in FIGS. 9A-9G, the mating locking features include a male latching feature 26010 on the connector 26000 and a female latching feature 26014 on the disposable housing assembly 26012.

In various embodiments, once the connector 26000 is attached to the disposable housing assembly 26012, the connector 26000 may only be removed when intended, i.e., the connector 26000 is maintained on the disposable housing assembly 26012 unless and until a user desires to remove the connector 26000. As discussed above, in some embodiments, the connector 26000 may be non-removably attached, however, in some embodiments; the connector 26000 may be removably attached.

While the connector 26000 is being attached to the disposable housing assembly 26012, the male latching feature 26010 is mated with the female latching feature 26014. In some embodiments, male and female latching features 26010, 26014 may work together to further stabilize the connection of the connector 26000 to the disposable housing assembly 26012. Additionally, in various embodiments, the male and female latching features 26010, 26014 may contribute to maintaining the plug 26002 in the exit of the disposable housing assembly 26012. Thus, in some embodiments, the male and female latching features 26010, 26014 may contribute to maintaining and/or enforcing both the insertion of the plug 26002 into the exit and/or maintaining and/or enforcing the position of the connector 26000 such that after the plug 26002 is inserted into the exit, the plug 26002 is maintained in the exit unless and until a user desires to remove the plug 26002 from the exit. Additionally, once the male and female latching features 26010, 26014 are mated, the plug 26002 is fully inserted and therefore, may, in some embodiments, serve as an indication that the plug 26002 has been fully inserted into the exit.

As discussed above, in the embodiment shown in FIGS. 9A-9G, the plug 26002 in the connector 26000 is slidably attached to the connector 26000 such that the plug 26002 may move forward or back and the connector tab portion 26008 may move independent from the plug 26002. This may be beneficial/desirable for many reasons, including, but not limited to, the mating of the luer (i.e., mating between the plug 26002 and the exit of the disposable housing assembly 26012) may be completed before the tab portion 26008 of the connector 26000 is rotated and attached to the disposable housing assembly 26012. Thus, the rotation of the tab portion 26008 about the plug 26002 is to connect the tab portion 26008 to the disposable housing assembly 26012, but the luer connection of the plug 26002 to the exit is completed by pushing the plug 26002 into the exit of the disposable housing assembly 26012. The plug 26002 being slidable within the tab portion 26008 of the connector 26000 is beneficial/desirable for many reasons, including, but not limited to, the depth in which the plug 26002 travels within the exit of the disposable housing assembly 26012 to form a luer connection may vary due to variances, for example, tolerances due to manufacturing. Thus, the plug 26002 may move deeper into the exit if the plug is smaller in comparison with the exit, or the exit is larger in comparison with the plug 26002; and the plug 26002 may form a connection shallower in the exit if the plug 26002 is large in comparison with the exit or the exit is smaller in comparison with the plug 26002. Thus, variances in tolerances will not prevent a luer connection from being made between the plug 26002 and the exit/disposable housing assembly 26012.

In some embodiments, the tab portion 26016 of the disposable housing portion 26012 may be shaped as shown in FIGS. 9E-9G. In some embodiments, the connector 26000, once attached to the disposable housing assembly 26012, may extend the tab portion 26016 of the disposable housing assembly 26012. In some embodiments, once the connector 26000 is attached to the disposable housing assembly 26012, the connector 26000 and tab portion 26016 may be flush and continuous.

In some embodiments, the connector 26000 may include an indent 26020, as shown. The indent 26020 may be shaped as shown or, in various embodiments, may be shaped and sized differently.

In various embodiments, the female latching feature 26014 on the disposable housing assembly 26012 may be located adjacent to the tab portion 26016. In some embodiments, for example, as shown in FIGS. 9E-9G, the female latching feature 26014 may be configured such that it receives the male latching feature 26010 on the connector 26000 from the top. Some embodiments may not include mating latching features 26010, 26014. In some embodiments, the location and/or orientation of the female latching feature 26014 may vary. In some embodiments, the location and/or orientation of the male latching feature 26010 may vary.

In some embodiments, the connector 26000 may be made from rigid plastic. In some embodiments, the locking ring feature and/or the latching features may be overmolded with a thin layer of compliant materials. In some embodiments, as discussed above, the plug 26002 may include an overmold of compliant material and/or be made from compliant material. In some embodiments, the locking ring feature and/or the latching feature may be made from compliant material. In embodiments where the male or female latching features 26010, 26014 include compliant material, use of compliant material may increase the "squish" between the male latching feature 26010 and the female latching feature 26014 and therefore, resulting in a highly compressed and/or tight fit between the male and female latching features 26010, 26014.

In some embodiments, the connector 26000 may include icons that indicate "locked" and "unlocked". Thus, in some embodiments, the "locked" and "unlocked" position may also be visually indicated to a user / patient using icons that may be molded, silk-screened, pad printed, injection molded, etched, printed and/ or cut-out, e.g., translucent cut-outs of icons, on the connector 26000. In some embodiments using translucent cut-outs, the tab portion 26016 of the disposable housing assembly 26012 may be a contrasting color to the connector 26000 for visually viewing the tab portion 26016 of the disposable housing assembly 26012 color through the cut-outs. Thus, the icons may indicate whether the reusable housing assembly is in a locked or unlocked relationship with the disposable housing assembly 26012. In various embodiments, the icons may be any form that may indicate "locked" and "unlocked", or a similar indication, to aid in the user / patient's understanding of the orientation/position between the reusable housing assembly and the disposable housing assembly 26012. In some embodiments, an arrow icon may also appear between the "locked" and "unlocked" icons.

Although not shown in FIGS. 9A-9G, in various embodiments, the reusable housing assembly may be any one or more of the various examples of the reusable housing assembly described herein, including, but not limited to, the reusable housing assembly shown and described above as 6004.

The plug 26002 may include any embodiment described herein, however, in some embodiments; the plug 26002 may be tapered and may either be rigid without any overmolding, be rigid with an overmold of elastomeric / compliant material or be made from elastomeric/compliant material. In various embodiments, the plug 26002 may include a seal, for example, in some embodiments; the plug 26002 may include one or more of: a radial seal and / or a face seal and /or both.

In some embodiments, tubing attaches to the connector 26000 as described above. In some embodiments, the tubing may attach to the connector 26000 and there may be a rigid plastic channel within the connector 26000 and through the plug 26002. In some embodiments, the tubing may extend into the connector 26000 and in some embodiments; the tubing may extend all the way through the connector 26000 and through the plug 26002. In some embodiments, the tubing may extend past the end of the plug 26002. Although not shown in FIGS. 9A-9G, the various embodiments of the connector may include any of the embodiments of the tubing and tubing connection as shown and described above with respect to various other embodiments of the connector.

In various embodiments, there is maintained a continuous flow lumen from the exit to the cannula (not shown). This may be desirable and/or beneficial for many reasons, including, but not limited to, minimizing and/or eliminating dead volume, minimizing priming volume and/or prevention of or minimizing the occurrence of air traps.

Some embodiments of the connector 26000 may also include a catch/catching feature 26018 on the opposite side as the male latching feature 26010. The catch feature 26018, when the connector 26000 is connected to the disposable housing assembly 26012, interferes with the disposable housing assembly 26012 and prevents the connector 26000 from rotating further. Thus, in some embodiments, the connector 26000 and the disposable housing assembly 26012 form an interference fit in at least one location. Together with the male and female latching features 26010, 26014, in some embodiments, once the connector 26000 is attached, the connector 26002 may be held in place by these mating features 26010, 26014.

The connector tab portion 26008 may include gripping features, however, in the some embodiments; the connector 26000 is sized such that a user may grip the connector 26000 for insertion/attachment with the disposable housing assembly 26012. Some embodiments including grip features, may include, but are not limited to, one or more of the following: a textured surface, bumps, lumps, protrusions or indentations in any size, shape and/or number.

In various embodiments of the connector 26000, once the connector 26000 is connected to the disposable housing assembly 26012, the reusable housing assembly 6004 (which may also be one or more of the various reusable housing assemblies described herein or incorporated herein by reference) may be connected/attached to the disposable housing assembly 26012 by being rotated about the disposable housing assembly 26012. As the reusable housing assembly is rotatably connected to the disposable housing assembly 26012, the locking ring and/ or nub 808, having a spring actuated tab 2980, on the reusable housing assembly 6004 may interact with the connector 26000, such that the rotation of the reusable housing assembly 6004 about the disposable housing assembly 26012 prevents the connector 26000 from being removed from the disposable housing assembly 26012. In some embodiments, once the reusable housing assembly 6004 is attached to the disposable housing assembly 26012, the spring plunger/tab in the nub 808 may be released, making a "click" sound. The "click" may also produce a tactile feedback that may be perceived by the user/patient. This tactile and audio feedback is indicative to the user that the reusable housing assembly 6004 is fully connected to the disposable housing assembly 26012, and, that the connector 26000 is connected such that it will be maintained connected to the disposable housing assembly 26012 until and unless the user wishes to remove the connector 26000. In some embodiments, the nub 808, having a spring actuated tab 2980, presses downward on the connector 26000, maintaining the connector 26000 in an attached position. In some embodiments, features in the locking ring interact with a locking ring feature on the connector 26000 and contribute to maintain the connector 26000 in position with respect to the disposable housing assembly 26012. In some embodiments, the tab portion 26008 of the connector 26000 may include an indent portion 26020 which may be configured to interact with the nub 808 and/or spring plunger/tab of the locking ring assembly of the reusable housing assembly 6004. In some embodiments, once the reusable housing assembly 6004 is attached to the disposable housing assembly 26012, the connector 26000 may not be removed from the disposable housing assembly 26012. Rather, in these embodiments, the reusable housing assembly 6004 must first be detached from the disposable housing assembly 26012 before the connector 26000 may be removed from the disposable housing assembly 26012. In some embodiments, once the connector 26000 is attached to the disposable housing assembly 26012, it may not be removed.

In FIGS. 9A-9G, the connector 26000 connects to the disposable housing assembly 26012 by the connector 26000 being rotated clockwise with respect to the disposable housing assembly 26012. In other embodiments, the various features described may be configured differently and the connector 26000 may be connected to the disposable housing assembly 26012 by the connector 26000 being rotated counter-clockwise with respect to the disposable housing assembly 26012.

In some embodiments, the disposable housing assembly 26012 may include a plug and the connector 26000 may include a plug receiver according to the various embodiment described herein. In some embodiments, the plug 26002 on the connector 26000 may be located in a different location and/or orientation than is shown herein. In various embodiments, the disposable housing assembly 26012 may include one or more mating features that correspond to one or more mating features on a connector 26000. In some embodiments, these mating features may be located and/or orientated differently than is shown herein.

The embodiment of the connector 26000 shown in FIGS. 9A-9G may vary in various embodiments and may include one or more features from any one or more embodiments of the connector and/or disposable housing assembly shown herein.

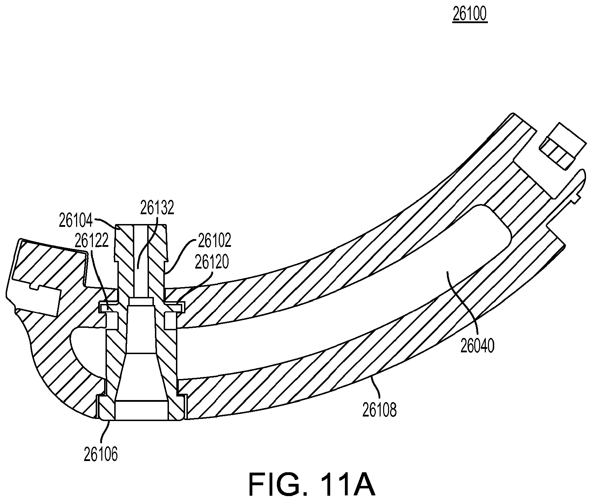

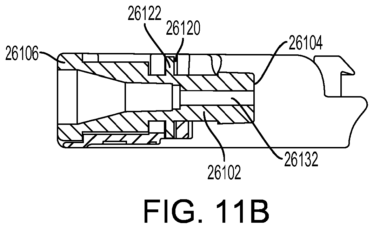

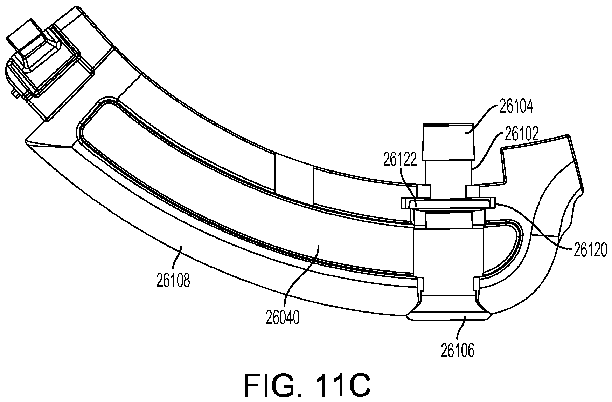

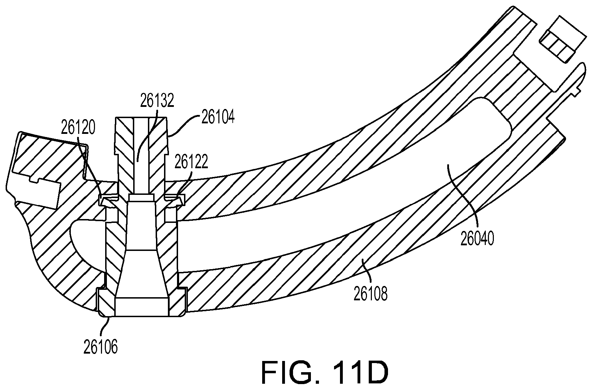

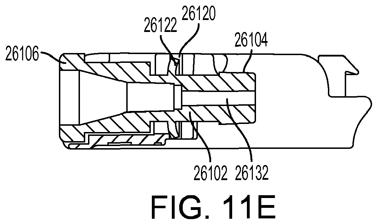

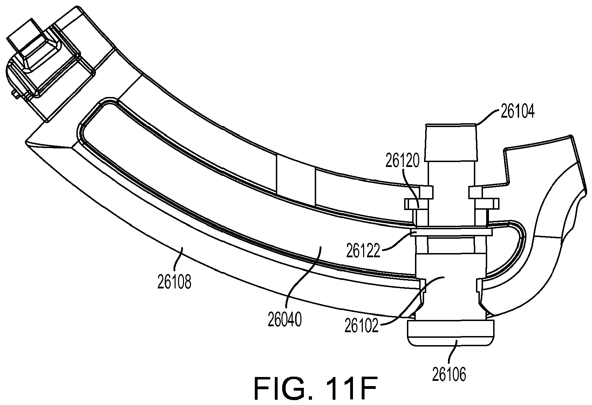

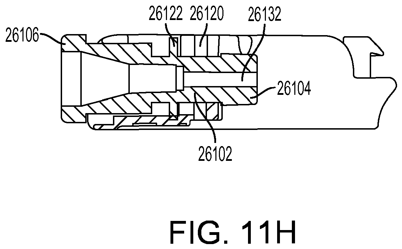

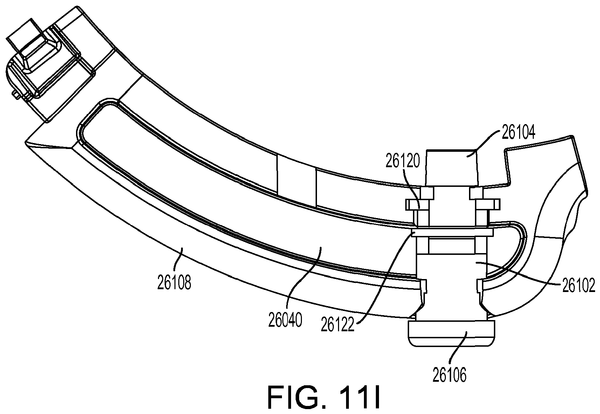

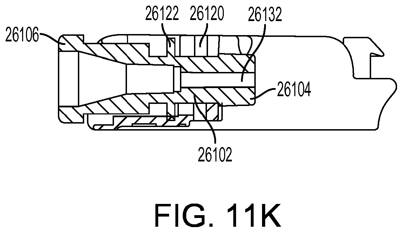

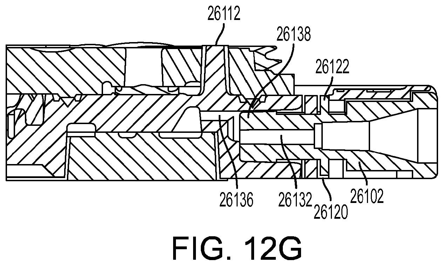

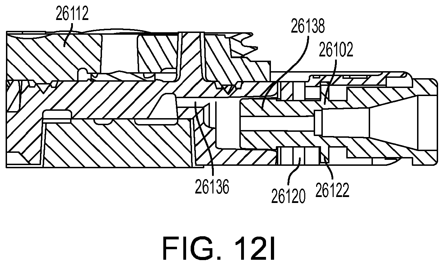

Referring now also to FIGS. 10A - 12I, some embodiments of the connector 26100 include a plug 26102 that is rigid and forms a luer connection with the exit of the disposable housing assembly 26112. In various embodiments, the plug 26102 may be tapered, and the percentage of tapering may vary. In some embodiments, the taper may be 6%, however, in various other embodiments; the taper may be greater or less than 6%. In some embodiments, including the one shown in FIGS. 10A - 10K, the plug 26102 is removably attached to the connector 26100 and, in addition, is slidably connected to the connector 26100. Additionally, in some embodiments, the plug 26102 may include a disc 26118 that, together with a slot 26120 control the movement of the plug 26102 with respect to the tab portion 26108 of the connector 26100. Thus, as shown in FIGS. 11A through 11J, the plug 26102 may slide from an initial position, shown in FIGS. 11A-11B and FIG. 12G, to a second position, shown in FIGS. 11C-262E and FIG. 12H, to a third position, shown in FIGS. 11F-11H, to a final position, shown in FIGS. 11I-11K and FIG. 12I. As may be seen from FIGS. 12G-12I, in the various positions of the plug as the plug moves from the initial position to the final position, the plug 26102 occupies less of the exit region 26138 of the disposable housing assembly 26112. As discussed above, this is due to the tolerance variations of the plug and/or the disposable housing assembly exit region 26138 or both. In various embodiments, the disc and slot may be configured wherein twice the force required to complete the luer connection is required to unseat the disc from the slot. This may be beneficial/desirable for many reasons, including, but not limited to, in various embodiments, if the disc is unseated from the slot, this ensures the luer connection has been made.

In embodiments, the plug 26102 is configured such that it includes at least one stop feature wherein the exit end portion 26104 of the plug 26102 includes a first stop feature, for example, a lip, that limits the travel of the plug 26104 in one direction. The plug 26102 tubing end portion 26106 in various embodiments also includes a second stop feature, for example, a lip, that limits the travel of the plug 26102 in the other direction. In various embodiments, the plug 26102 may be made from any material, for example, but not limited to, TERLUX^{®} and/or another ABS material. Although not shown in the embodiments shown in FIGS. 10A-12I, the plug 26102 includes a length of tubing attached to the tubing end portion 26106, which tubing and attachment may be any one or more of the various embodiments described in more detail above with respect to various embodiments of the plug and the connector. The length of tubing may be any length desired, including, but not limited to, any length between 1 inch and 10 feet, and in some embodiments may be either 4 inches or 23 inches, however, in various embodiments, the length may be greater or less than 4 inches or 23 inches.

In various embodiments, the connector 26100 includes a tab portion 26108 including a mating locking feature 26110, which may be, in various embodiments, the mating locking feature described above with respect to various other embodiments of the connector. In various embodiments, the mating locking feature 26110 is configured to mate with a corresponding mating locking feature 26114 on the disposable housing assembly 26112. In various embodiments, the mating locking feature 26110 on the connector 26100 including a hook that latches to the corresponding mating locking feature 26114 on the disposable housing assembly 26112.

In various embodiments, the plug 26102 of the connector 26100 is inserted into the disposable housing assembly 26112 with the tab 26108 portion pointing upward. Once the plug 26102 portion is inside the disposable housing assembly 26112, the tab 26108 portion may be rotated about the plug 26102 to rest above the disposable housing assembly 26112 adjacent to the exit (i.e., rest on the tab portion 26116 on the disposable housing assembly 26112). In some embodiments, mating locking features 26110, 26114 may be included on the tab portion 26108 of the connector 26100 and the disposable housing assembly 26112, respectively, such that the connector 26100 is held in place before the reusable housing assembly 6004 is attached to the disposable housing assembly 26112. In some embodiments, the mating locking features 26110, 26114 may include, but are not limited to, snap buttons and / or catch features. In some embodiments, a hook or other feature may be located on the opposite end of the tab 26108 portion of the connector 26100 such that it loops over the end of the tab portion 26116 of the disposable housing assembly 26112 and maintains the position of the connector 26100.

Still referring also to FIGS. 10A-12I, the connector 26100 may include a tab portion 26108, a catch feature 26118, a latching feature 26110, and a plug 26102. In some embodiments, the tab portion 26108 of the connector 26100 may include mating locking features that interact with corresponding features in the disposable housing assembly 26112. In some embodiments, the tab portion 26108 may include features that interact with other portions of the disposable housing assembly 26112. As shown, in some embodiments, the connector 26100 may include a male latching feature 26110 on the end of the tab portion 26108 of the connector 26100. In some embodiments, the latching feature 26110 may mate with a corresponding latching feature (a female latching feature 26114) on the disposable housing assembly 26112. In some embodiments, the latch may be a removably secure fit, and / or a snap fit and/or a loose snap fit and in some embodiments may include a spring latch portion. In various other embodiments, the shape and size of the connector 26100 may vary, and/or, in various other embodiments, other types of features such as mating locking features may be used, which include, but are not limited to, latches, catches, snap fits, adhesives, and other mechanisms for securing a connector 26100 to the tab 26116 of the disposable housing assembly 26112.

In some embodiments, the connector 26100 may include a locking ring feature on the underside. The locking ring feature in some embodiments may be tapered and/or in some embodiments, the locking ring feature may be at least slightly curved. In some embodiments, the locking ring feature may interact with the locking ring of the reusable housing assembly and may act together with the locking ring to secure the connector to the disposable housing assembly.

As discussed above, in various embodiments, the plug 26102 of the connector 26100 is inserted into the exit of the disposable housing assembly 26112 with the tab portion 26108 pointing in the general upward direction. Once the plug 26102 is inside the exit, the tab portion 26108 of the connector 26100 may be rotated to rest adjacent to the tab portion 26116 of the disposable housing assembly 26112. In some embodiments, mating locking features may be included on the tab portion 26108 of the connector 26100 and the disposable housing assembly 26112 such that the connector 26100 is held in place before the reusable housing assembly is attached to the disposable housing assembly 26112. In some embodiments, the mating locking features may include, but are not limited to, post and opening, snap, buttons, latch features and / or catch features. In some embodiments, a hook or other feature may be located on the opposite end of the tab portion 26108 of the connector 26100 such that it loops over the end of the tab portion 26116 of the disposable housing assembly 26112 and maintains the position of the connector 26100. In the embodiment shown in FIGS. 10A-12I, the mating locking features include a male latching feature 26110 on the connector 26100 and a female latching feature 26114 on the disposable housing assembly 26112.

In various embodiments, once the connector 26100 is attached to the disposable housing assembly 26112, the connector 26100 may only be removed when intended, i.e., the connector 26100 is maintained on the disposable housing assembly 26112 unless and until a user desires to remove the connector 26100. As discussed above, in some embodiments, the connector 26100 may be non-removably attached, however, in some embodiments; the connector 26100 may be removably attached.

While the connector 26100 is being attached to the disposable housing assembly 26112, the male latching feature 26110 is mated with the female latching feature 26114. In some embodiments, male and female latching features 26110, 26114 may work together to further stabilize the connection of the connector 26100 to the disposable housing assembly 26112. Additionally, in various embodiments, the male and female latching features 26110, 26114 may contribute to maintaining the plug 26102 in the exit of the disposable housing assembly 26112. Thus, in some embodiments, the male and female latching features 26110, 26114 may contribute to maintaining and/or enforcing both the insertion of the plug 26102 into the exit and/or maintaining and/or enforcing the position of the connector 26100 such that after the plug 26102 is inserted into the exit, the plug 26102 is maintained in the exit unless and until a user desires to remove the plug 26102 from the exit. Additionally, once the male and female latching features 26110, 26114 are mated, the plug 26102 is fully inserted and therefore, may, in some embodiments, serve as an indication that the plug 26102 has been fully inserted into the exit.

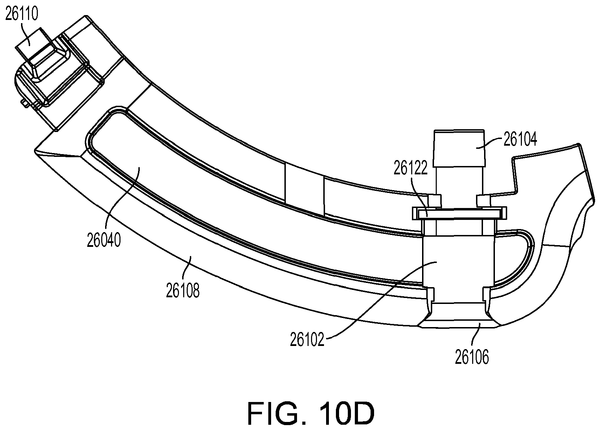

As discussed above, in the embodiment shown in FIGS. 10A-12I, the plug 26102 in the connector 26100 is slidably attached to the connector 26100 such that the plug 26102 may move forward or back and the connector tab portion 26108 may move independent from the plug 26002. In addition, in this embodiments of the slidably attached connector plug 26102, the plug 26102 is biased forward with respect to the tab portion 26108 of the connector 26100. This may be beneficial/desirable for many reasons, including, but not limited to, the mating of the luer (i.e., mating between the post 26002 and the exit of the disposable housing assembly 26012) may be completed before the tab portion 26008 of the connector 26000 is rotated and attached to the disposable housing assembly 26012. Thus, the rotation of the tab portion 26008 about the plug 26002 is to connect the tab portion 26008 to the disposable housing assembly 26012, but the luer connection of the plug 26002 to the exit is completed by pushing the plug 26002 into the exit of the disposable housing assembly 26012. The plug 26002 being slidable within the tab portion 26008 of the connector 26000 is beneficial/desirable for many reasons, including, but not limited to, the depth in which the plug 26002 travels within the exit of the disposable housing assembly 26012 to form a luer connection may vary due to variances, for example, tolerances due to manufacturing. Thus, the plug 26002 may move deeper into the exit if the plug is smaller in comparison with the exit, or the exit is large in comparison with the plug 26002 and the plug 26002 may form a connection shallower in the exit if the plug 26002 is large in comparison with the exit or the exit is smaller in comparison with the plug 26002. Thus, variances in tolerances will not prevent a luer connection from being made between the plug 26002 and the exit/disposable housing assembly 26012. Also, the embodiment shown in FIGS. 10A-12I may be beneficial/desirable for many reasons, including, but not limited to, ensuring that the luer connection is successful between the plug 26102 and the disposable housing assembly 26112 and that the plug 26102 is prevented from moving away from the disposable housing assembly 26112 unless tolerances dictate that the plug 26102 moves away from the disposable housing assembly 26112 in order to form the luer connection. Thus, the disc 26122, in the initial position with respect to the tab portion 26108 (shown, for example, in FIGS. 10D-10H) is in the slot 26120 and is therefore prevented from sliding either backwards (away from the disposable housing assembly 26112) with respect to the tab portion 26108 unless tolerances between the plug 26102 and the exit portion of the disposable housing assembly are such that the disc moves away from the disposable housing assembly.

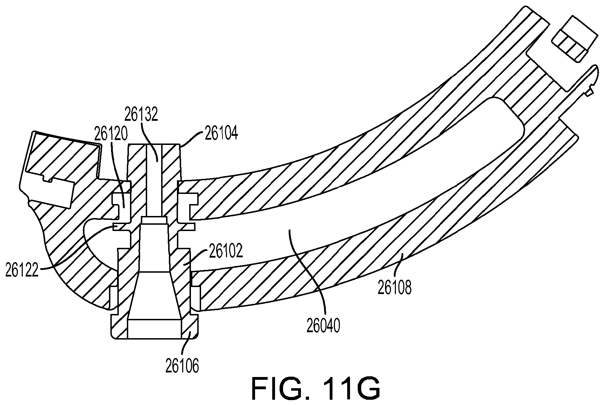

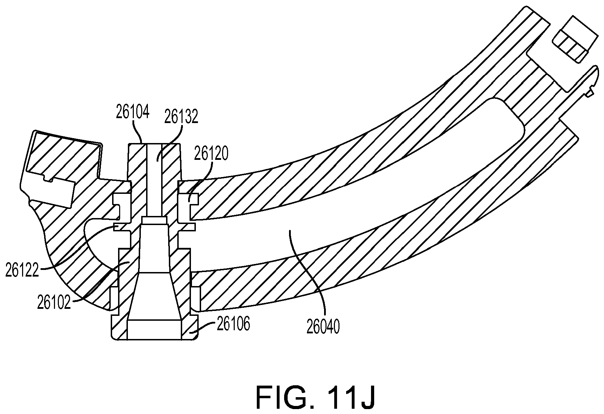

Thus, still referring to FIGS. 10A-12I, the plug 26102 is inserted into the exit portion of the disposable housing assembly and if, due to tolerances, the plug 26102 is not fully inside the exit, additional force applied to the plug 26102 (via the tab portion 26108 of the connector 26100) bends the disc 26122 such that the plug 26102 moves away from the disposable housing assembly 26112. This moves the plug 26102 from the initial position to a second position. The second position may be seen in FIGS. 11A-11C. If the plug 26102 is still not fully inside the exit, additional force applied to the plug 26102 detaches the disc 26122 from the slot 26120 and the plug 26102 slides further away from the disposable housing assembly 26112. This moves the plug 26102 from the second position to the third position. The third position may be seen in FIGS. 11G-11I. If the plug 26102 is still not fully inside the exit, additional force applied to the plug 26102 causes the plug 26102 to slide further away from the disposable housing assembly 26112. This moves the plug 26102 from the third position to the final position. The final position may be seen in FIGS. 11J- 11K. As may be seen in FIGS. 11J-11K, the exit end portion 26104 of the plug 26102 includes a feature, for example, a lip, that limits the travel of the plug 26102 in the direction moving away from the disposable housing assembly 26112. As may be seen in FIGS. 11J-11K, once the exit end portion 26104 reaches the tab portion 26108 of the connector 26100, the plug 26102 is prevented from moving further away from the disposable housing assembly 26112 and is in the final position with respect to the tab potion 26108 of the connector 26100.

Referring now also to FIGS. 10A-12I, in some embodiments, the tab portion 26116 of the disposable housing portion 26112 may be shaped as shown. In some embodiments, the connector 26100, once attached to the disposable housing assembly 26112, may extend the tab portion 26116 of the disposable housing assembly 26112. In some embodiments, once the connector 26100 is attached to the disposable housing assembly 26112, the connector 26100 and tab portion 26116 may be flush and continuous.

In some embodiments, the connector 26100 may include an indent 26124, as shown. The indent 26124 may be shaped as shown or, in various embodiments, may be shaped and sized differently.

In various embodiments, the female latching feature 26114 on the disposable housing assembly 26112 may be located adjacent to the tab portion 26116. In some embodiments, for example, as shown in FIGS. 12A-12C, the female latching feature 26114 may be configured such that it receives the male latching feature 26110 on the connector 26100 from the top. Some embodiments may not include mating latching features 26110, 26114. In some embodiments, the location and/or orientation of the female latching feature 26114 may vary. In some embodiments, the location and/or orientation of the male latching feature 26110 may vary.

In some embodiments, the connector 26100 may be made from rigid plastic. In some embodiments, the locking ring feature and/or the latching features may be overmolded with a thin layer of compliant materials. In some embodiments, as discussed above, the plug 26102 may include an overmold of compliant material and/or be made from compliant material. In some embodiments, the locking ring feature and/or the latching feature may be made from compliant material. In embodiments where the male or female latching features 26110, 26114 include compliant material, use of compliant material may increase the "squish" between the male latching feature 26110 and the female latching feature 26114 and therefore, resulting in a highly compressed and/or tight fit between the male and female latching features 26110, 26114.

Referring now also to FIGS. 10A-12I, in some embodiments, the connector 26100 may include icons 26126, 26128 that indicate "locked" (26126) and "unlocked" (26128), similar to those shown and described above. Thus, in some embodiments, the "locked" and "unlocked" position may also be visually indicated to a user / patient using icons 26126, 26128 that may be molded, silk-screened, pad printed, injection molded, etched, printed and/ or cut-out, e.g., translucent cut-outs of icons, on the connector 26000. In some embodiments using translucent cut-outs, the tab portion 26116 of the disposable housing assembly 26112 may be a contrasting color to the connector 26100 for visually viewing the tab portion 26116 of the disposable housing assembly 26112 color through the cut-outs. Thus, the icons 26126, 26128 may indicate whether the reusable housing assembly 6004 is in a locked or unlocked relationship with the disposable housing assembly 26112. In various embodiments, the icons 26126, 26128 may be any form that may indicate "locked" and "unlocked", or a similar indication, to aid in the user / patient's understanding of the orientation/position between the reusable housing assembly 6004 and the disposable housing assembly 26012. In some embodiments, an arrow icon 26130 may also appear between the locked icon 26126 and unlocked icon 26128.

Although not shown in FIGS. 10A-12I, in various embodiments, the reusable housing assembly may be any one or more of the various embodiments of the reusable housing assembly described herein, including, but not limited to, the reusable housing assembly described above as 6004.

The plug 26102 may include any embodiment described herein, however, in some embodiments; the plug 26102 may be tapered and may either be rigid without any overmolding, be rigid with an overmold of elastomeric / compliant material or be made from elastomeric/compliant material. In various embodiments, the plug 26102 may include a seal, for example, in some embodiments, the plug 26102 may include a radial seal, and in some embodiments, the plug 26102 may include a face seal and in some embodiments, the plug 26102 may include a radial and a face seal. In some embodiments, the plug 26102 may include one or more seals, including, but not limited to, a face seal and/or a radial seal.

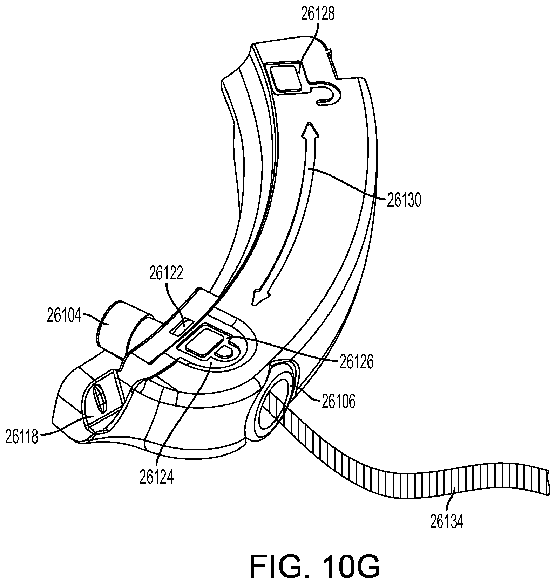

In some embodiments, tubing 26134 attaches to the connector 26100 as described above and as shown in FIG. 10G. In some embodiments, the tubing 26134 may attach to the connector 26100 and there may be a rigid plastic channel 26132 within the connector 26100 and through the plug 26102. In some embodiments, the tubing 26134 may extend into the connector 26100 and in some embodiments; the tubing 26134 may extend all the way through the connector 26200 and through the plug 26202. In some embodiments, the tubing 26134 may extend past the end of the plug 26102. Although not shown in FIGS. 10A-12I, the various embodiments of the connector 26100 may include any of the embodiments of the tubing and tubing connection as shown and described above with respect to various other embodiments of the connector.

In various embodiments, there is maintained a continuous flow lumen from the exit of the disposable housing assemby to the cannula (not shown). This may be desirable and/or beneficial for many reasons, including, but not limited to, minimizing and/or eliminating dead volume, minimizing priming volume and/or prevention of or minimizing the occurrence of air traps. As may be seen in FIGS. 12G-12I, once the plug 26102 is connected to the disposable housing assembly 26112, and a luer connection is made between the plug 26102 and the exit of the disposable housing assembly 26112, the fluid line 26136 within the disposable housing assembly 26102 is in fluid and continuous connection with the rigid plastic channel 26132 in the plug 26102. Where tubing is attached to the plug 26102, the fluid line 26136 within the disposable housing assembly 26102 is in fluid and continuous connection with the rigid plastic channel 26132 in the plug 26102 and the tubing.

Referring still also to FIGS. 10A- 12I, some embodiments of the connector 26100 may also include a catch/catching feature 26118 on the opposite side as the male latching feature 26110. The catch feature 26118, when the connector 26100 is connected to the disposable housing assembly 26112, interferes with the disposable housing assembly 26112 and prevents the connector 26100 from rotating further. Thus, in some embodiments, the connector 26100 and the disposable housing assembly 26112 form an interference fit in at least one location. Together with the male and female latching features 26110, 26114, in some embodiments, once the connector 26100 is attached, the connector 26102 may be held in place by these mating features 26110, 26114. The catch feature 26118 in various embodiments also aids in catching the disposable housing assembly and the ramp portion of the catch feature 26118 assists in pulling the connector 26100 towards the disposable housing assembly 26112.

The connector tab portion 26108 may include gripping features, however, in the some embodiments; the connector 26100 is sized such that a user may grip the connector 26100 for insertion/attachment with the disposable housing assembly 26112. Some embodiments including grip features, may include, but are not limited to, one or more of the following: a textured surface, bumps, lumps, protrusions or indentations in any size, shape and/or number.

In various embodiments of the connector 26100, once the connector 26100 is connected to the disposable housing assembly 26112, the reusable housing assembly 6004 (which may also be one or more of the various reusable housing assemblies described herein or incorporated herein by reference) may be connected/attached to the disposable housing assembly 26112 by being rotated about the disposable housing assembly 26112. As the reusable housing assembly is rotatably connected to the disposable housing assembly 26112, the locking ring and/ or nub 808, having a spring actuated tab 2980, on the reusable housing assembly 6004 may interact with the connector 26100, such that the rotation of the reusable housing assembly 6004 about the disposable housing assembly 26112 prevents the connector 26100 from being removed from the disposable housing assembly 26112. In some embodiments, once the reusable housing assembly 6004 is attached to the disposable housing assembly 26112, the spring plunger/tab in the nub 808 may be released, making a "click" sound. The "click" may also produce a tactile feedback that may be perceived by the user/patient. This tactile and audio feedback is indicative to the user that the reusable housing assembly 6004 is fully connected to the disposable housing assembly 26112, and, that the connector 26100 is connected such that it will be maintained connected to the disposable housing assembly 26112 until and unless the user wishes to remove the connector 26100. In some embodiments, the nub 808, having a spring actuated tab 2980, presses downward on the connector 26100, maintaining the connector 26100 in an attached position. In some embodiments, features in the locking ring interact with a locking ring feature on the connector 26100 and contribute to maintain the connector 26100 in position with respect to the disposable housing assembly 26112. In some embodiments, the tab portion 26108 of the connector 26100 may include an indent portion 26124 which may be configured to interact with the nub 808 and/or spring plunger/tab of the locking ring assembly of the reusable housing assembly 6004. In some embodiments, once the reusable housing assembly 6004 is attached to the disposable housing assembly 26112, the connector 26100 may not be removed from the disposable housing assembly 26112. Rather, in these embodiments, the reusable housing assembly 6004 must first be detached from the disposable housing assembly 26112 before the connector 26100 may be removed from the disposable housing assembly 26112. In some embodiments, once the connector 26100 is attached to the disposable housing assembly 26112, it may not be removed.

In the embodiment shown in FIGS. 10A-12I, the connector 26100 connects to the disposable housing assembly 26112 by the connector 26100 being rotated clockwise with respect to the disposable housing assembly 26112. In other embodiments, the various features described may be configured differently and the connector 26100 may be connected to the disposable housing assembly 26112 by the connector 26100 being rotated counter-clockwise with respect to the disposable housing assembly 26112. In various embodiments, the plug 26102 remains stationary as the connector tab portion 26102 is rotated about the plug 26102. However, in some embodiments, the plug 26102 may be rotated together with the connector 26100.

In some embodiments, the disposable housing assembly may include a plug and the connector may include a plug receiver according to the various embodiment described herein. In some embodiments, the plug 26102 on the connector 26100 may be located in a different location and/or orientation than is shown herein. In various embodiments, the disposable housing assembly 26112 may include one or more mating features that correspond to one or more mating features on a connector 26100. In some embodiments, these mating features may be located and/or orientated differently than is shown herein.

The embodiment of the connector 26100 shown in FIGS. 10A-12I may vary in various embodiments and may include one or more features from any one or more embodiments of the connector and/or disposable housing assembly shown herein.

Referring now also to FIGS. 9A-11I, in various embodiments, as described herein, the plug may form a luer connection with the exit of the disposable housing assembly. In various embodiments, the luer connection may be formed between the plug and disposable housing assembly by the plug being inserted into the exit of the disposable housing assembly and in some embodiments, the luer connection is formed between the plug being inserted in the exit of the disposable housing assembly and upon connection of the tab portion of the connector to the disposable housing assembly. As discussed above, in some embodiments the catch feature aids in completing the seal between the plug and the disposable housing assembly by pushing the plug further into the exit of the disposable housing assembly. Therefore, the luer connection may be formed without any rotation of the plug within the disposable housing assembly/exit of the disposable housing assembly. In various embodiments, therefore, the luer connection may be formed between the plug and disposable housing assembly before the tab portion of the connector is rotated about the plug and connected to the disposable housing assembly. This may be beneficial/desirable for many reasons, including but not limited to, forming a luer connection without rotation and then independently rotating a portion attached to the plug prevents the plug from breaking, which would likely occur if the plug were rotated together with the tab portion of the connector. This is beneficial/desirable for many reasons, including but not limited to, forming a luer connection without rotation allows for movement of other portions of an assembly that are not involved in the luer connection whilst the luer connection is maintained. Therefore, in some embodiments, the connector shown and described in, for example, FIGS. 9A-11I, is an assembly including a luer connection between one or more first portions and a second portion that may rotate independent of the luer connection first portion(s). This is beneficial/desirable for many reasons, including, but not limited to, where the tab portion of the connector could not rotate independent of the plug, once the luer binds, the motion of the tab portion would have to stop and would not rotate sufficiently for the latching features to meet and the connector to attach to the disposable housing assembly. Where the connector is not rotated sufficiently, the reusable housing assembly would not be able to be attached to the disposable housing assembly. The embodiments described with respect to, for example, FIGS. 9A-11I, may also be beneficial/desirable for many reasons, including but not limited to, the plug and the tab portion of the connector may be made from different materials as they are separate parts and the absence of o-rings, radial seals, facial seals, etc. is beneifical/desirable for many reasons, including, but not limited to, seals may have parting lines and other manufacturing introduced features that may degrade the seal, and luer connections are more reliable seals.

Still referring to FIGS. 9A-12I, in various embodiments, the connector may be connected to tubing (for example, as showm in FIG. 10G) and the tubing may be connected to a cannula assembly, such as one described above and shown in, for example, FIGS. 236G and 236H, however, various embodiments of a cannula assembly may be used together with the connectors described herein.

Still referring also to FIGS. 9A-12I, in some embodiments, the connector may include a "core" 26040 on the bottom. The core 26040 may be an opening in the connector. In some embodiments, an identification tag may be located in the core 26040, however, in various other embodiments, the identification tag may be located in another location on the connector (and in some embodiments, the identification tag may be located on the disposable housing assembly and/or on the cannula assembly). In some embodiments it may be desired to include a method of the system identifying the connector (and/or disposable housing assembly and/or cannula assembly) and/or a device for the system to identify the connector. In some embodiments, the identification tag may be an RFID tag. In some embodiments, the identification tag may be a near-field communication ("NFC") readable RFID tag. In some embodiments, the identification tag may be a 2D or 3D bar code. In some embodiments, the identification tag may be a QR code.

In various embodiments, either before the connector is connected to the disposable housing assembly or after the reusable housing assembly has been connected to the disposable housing assembly, the reusable housing assembly 6004 and/or the remote control unit may read the identification tag. The identification tag 6092 may include various information, including, but not limited to, one or more of the following: unique identification number, date of manufacture, date of expiration, part number, lot number, and/or date of sale. Once the system reads the identification tag, the system may recognize the connector by its unique identification number or other, and thus, be able to recognize that the connector is either a new connector 6010, i.e., has not been used previously, or is an old connector, i.e., the connector has been used previously. In some embodiments, once the connector is attached, the system may start a timer and the infusion pump may alert/alarm and/or turn off after a predetermined period of time where the connector has been used by the system. In some embodiments, this predetermined time may be the maximum time that the manufacturer recommends using the connector and/or cannula assembly attached to the connector. In some embodiments, this predetermined time may be the maximum time that the manufacturer recommends using the disposable housing assembly.

In some embodiments, the information conveyed by the identification tag may include information to authenticate the connector, i.e., to ensure that the connector is not a counterfeit part. In some embodiments, the remote control unit and/or reusable housing assembly /pump may include a listing of authentic identification information such that the system may "authenticate" the connector based on information received from the manufacturer.

In various embodiments, the identification tag may be a "peel and stick" RFID tag, which may be attached to the connector, for example, in the core 26040 area of the connector. In some embodiments, the identification tag may be glued and/or potted and/or another part may be pressed over the identification tag to sandwich the identification tag. In some embodiments, the identification tag may be taped to the connector. In some embodiments, where the identification tag is a bar code and/or QR code, the identification tag may be embossed, printed and/or molded onto the connector.

In some embodiments, the identification tag may be an RFID tag. In some embodiments, the RFID tag may be a tag that includes an antenna and chip. In some embodiments, the connector may be manufactured with a conductive ring and a chip could be electrically connected to the conductive ring. In some embodiments, the RFID tag could be on the label which could include additional information, for example, but not limited to, text, for example, part number, label, instructions, expiration date.

In some embodiments, the reusable housing assembly may include hardware to read the identification tag, which may include an RFID chip and/or a bar code, for example, 2D or 3D, or a QR code. For example, in some embodiments, the hardware may be an antenna which may be a copper trace on a board. In some embodiments the hardware may include a camera and/or a bar code scanner. In some embodiments, the remote control until may include hardware to read the identification tag, for example, in some embodiments, the remote control until may include an RFID transceiver and/or a near-field communication transceiver which may read the identification tag in those embodiments where the identification tag is an RFID tag. In some embodiments, the RFID tag may be one that may be read by an NFC transceiver. In some embodiments the remote control until may include a camera and/or a bar code scanner.

In some embodiments, using NFC may be desirable for many reasons, including, but not limited to, the reusable housing assembly and the remote control unit could both include NFC transceivers and the reusable housing assembly and the remote control unit may be paired in this fashion. This provides that the reusable housing assembly and the remote control unit come into physical contact with one another. This may be beneficial for many reasons including, but not limited to, the devices being paired are both in control of the user/patient and therefore, this may provide additional localized security when pairing the devices, i.e., to ensure that a non-intended device is not paired with either the remote control unit or the reusable housing assembly.

In some embodiments, the cannula assembly, connector and/or the disposable housing assembly may include identification tags and may include one or more of the features discussed above with respect to identification tags and/or may be used in one or more of the methods discussed above.

In various embodiments, the reusable housing assembly may begin pumping fluid only after either the reusable housing assembly or the remote control unit has received the information from the one or more identification tags in the system (e.g., disposable housing assembly, cannula assembly and/or connector) and has determined that the information received is acceptable. For example, in some embodiments, if the information received does not convey to the system that a "new" and/or "acceptable" (i.e., is not expired, has not been recalled) connector and/or disposable housing assembly and/or cannula assembly is being connected to the system, then the system may not pump fluid and or may alert/alarm.

Various embodiments of the connector may be made from any color desired. In some embodiments, the connector may be made from a different color than the disposable housing assembly. In some embodiments, the connector may be made from the same color as the disposable housing assembly.

In some embodiments the connector, after being connected / attached to the disposable housing assembly, may not be removed from the disposable housing assembly. In some embodiments, once connected / attached to the disposable housing assembly, it may be difficult for a user to remove the connector from the disposable housing assembly. In some embodiments, this may be beneficial and/or desirable for once the tubing has been primed; the tubing is full of fluid, which, in some embodiments, may include a therapeutic fluid. If the connector were removed from the disposable housing assembly, and a cannula assembly, in fluid communication with the connector, was already inserted into a user, and if the connector were to be elevated such that it was higher than the cannula assembly, the fluid in the tubing may be delivered through the cannula assembly to the user. In some circumstances, this may not be desirable for many reasons, including, but not limited to, unintentional delivery of a volume of fluid. Therefore, prevention of this circumstance may be desirable.

Referring now also to FIGS. 15A - 15B, an embodiment of a fill adapter 9000 is shown. In this embodiment, the fill adapter 9000 includes guide features 9032 to guide the button assemblies.

Referring now also to FIGS. 16A - 16F, another embodiment of the filling aid 9004 is shown. In this embodiment, the filling aid 9004 is configured to receive a filling syringe (not shown, shown as 9062 in FIGS. 17A- 17B), which includes a filling needle (not shown, shown as 9014 in FIG. 17A) attached to the filling syringe 9062. The filling aid 9004 in this embodiment includes a filling needle cradle 9016

In the embodiment shown in FIGS. 16A - 16F, the needle housing 9038 includes needle housing locking features 9066 which interact with the filling needle cradle 9016 to lock the filling needle cradle 9016 either in a starting or filling position. The filling needle cradle 9016 may be unlocked from its position by rotating the filling needle cradle 9016 with respect to the needle housing 9038.

In various embodiments, the filling needle cradle 9016 includes filling needle cradle ribs 9060 which capture the filling syringe 9062 and maintain the filling syringe 9062 in an attached position with respect to the filling needle cradle 9016.

In various embodiments, the filling needle cradle 9016 also includes a stop feature 9068 which limits the distance the filling syringe 9062 may travel within the filling needle cradle 9016. However, the filling needle 9014 itself may travel through the stop feature 9068.

Referring now also to FIGS. 17A- 20, in practice, the filling syringe 9062 is attached to the filling aid 9004 by pressing the filling syringe 9062 through the opening of the filling aid 9004. The filling needle 9014 cap may then be removed. Next, the filling aid 9004 is attached to a vial of fluid 9080 by using the end tabs of the needle housing. The vial of fluid may contain e.g., therapeutic fluid, e.g., insulin. The filling needle 9014 may penetrate the septum 9082 and the fluid may flow from the vial 9080 to the filling syringe 9062 by way of the filling needle 9014. Once a desired volume of fluid flows from the vial 9080 to the filling syringe 9062, the vial 9080 may be removed from the needle housing 9038 , e.g., by exerting opposing force onto the needle housing 9038 and the vial 9080 so as to disconnect the end tabs of the needle housing 9038 from the vial 9080.

These embodiments may be advantageous, beneficial and/or desirable for many reasons including that the filling needle 9014 remains inside the needle housing 9038 while the filling syringe 9062 is being filled by the fluid from the vial 9080. Therefore, the likelihood of unintentional needle pricks and/or contamination is minimized and/or reduced. Thus, in various embodiments, the filling needle cradle 9016 remains in the starting position (see FIGS. 17A and 17B ) while the filling aid 9004 is attached to a vial 9080.

Although the embodiments are described above with respect to a "vial" in some embodiments, the filling aid 9004 may be used in conjunction with any source of fluid, which may include, but is not limited to, a bag of fluid.

In various embodiments, once the filling syringe 9062 is removed from the vial 9080, the filling syringe 9062, still attached to the filling aid 9004 may then be connected to the filling aid base 9046. In some embodiments, connection of the filling aid 9004 to the filling aid base 9046 may be made by sliding the filling aid 9004 over the filling aid base 9046. Thus, in various embodiments, the filling aid base 9046 may include a smaller diameter than the filling aid 9004 such that the filling aid base 9046 may be received by the filling aid 9004.

In some embodiments, the connection between the filling aid 9004 and the filling aid base 9046 may include the filling aid 9004 sliding onto the filling aid base 9046 and then the filling aid 9004 being rotated with respect to the filling aid base 9046. In some embodiments, the rotation may attach or lock the filling aid 9004 to the filling aid base 9046 such that the filling aid 9004 may not be removed from the filling aid base 9046 unless the filling aid 9004 is rotated back. Therefore, in some embodiments, the filling aid base 9046 may remain connected the filling aid 9004 while the reservoir is being filled, but may be removed from the each other once the filling is completed. Thus, in some embodiments, the filling aid 9004 may include a locked position with respect to the filling aid base 9046 and may include an unlocked position with respect to the filling aid base 9046.

In some embodiments, when the filling aid 9004 is connected to the filling aid base 9046, the filling needle cradle 9016 may be in a starting position. The starting position means that the filling needle 9014 remains fully inside the filling needle cradle 9016 and therefore, although the filling aid 9004 is attached to the filling needle base 9046, the filling needle 9014 has not pierced the septum of the reservoir. Referring now also to FIG. 19, the filling needle cradle 9016 is in the starting position.

In some embodiments, the needle housing 9038 includes needle housing locking features 9066. In various embodiments, when the filling needle cradle 9016 is in the starting position, the needle housing locking features 9066 are in the starting position and the relationship between the needle housing locking features 9066 and the filling needle cradle 9016 is such that the needle housing locking features 9066 anchor the filling needle cradle 9016 in the starting position.

As the needle housing 9038 is attached to the filling aid base 9046, to unlock the needle housing locking features 9066, the filling needle cradle 9016 may be rotated and unlocked. The filling needle cradle 9016 may then slide towards the filling aid base 9046, sliding with respect to the needle housing 9038. Thus, the filling needle cradle 9016 is prevented from sliding with respect to the needle housing 9038 until and unless it is rotated. Therefore, the filling needle 9014 is maintained within the needle housing 9038 until the filling aid 9004 is attached to the filling aid base 9046. This embodiment may be beneficial for many reasons, including, but not limited to, prevention of needle contamination and/or damage and / or prevention of unintentional needle sticks, etc.

In various embodiments, to advance the filling needle 9014 towards the septum of the disposable housing assembly 9002, the filling needle cradle 9016 needs to be rotated, unlocking with respect to the needle housing locking features 9066This, in various embodiments, advances the filling needle cradle 9016 towards the septum such that the filling needle cradle 9016 is in the filling position. In this position, the filling needle 9014 pierces the septum (see FIG. 20)

In various embodiments locking the filling aid 9004 from rotation with respect to the filling aid base 9046 when the filling needle cradle 9016 is in the filling position may be desirable for many reasons, including, but not limited to, once the filling needle 9014 pierces the septum, rotation of the filling aid 9004 with respect to the filing aid base 9046 may cause damage to the septum and/or the reservoir and/or cause leakage.

Once the filling needle 9014 pierces the septum of the disposable housing assembly 9002, the plunger portion of the filling syringe 9062 may be advanced to provide flow of fluid from the filling syringe 9062 to the reservoir 908.

In various embodiments, the disposable housing assembly 9002 may have a tubing assembly 9090 and/or a tubing assembly 9090 connected to a cannula assembly 9092 (which, in some embodiments, may be a connection to a cannula assembly) attached.

In various embodiments, the fill adapter may be a reusable portion and the filling aid and filling syringe may be disposable/limited (e.g., one time) use portions. In some embodiments, the disposable housing assembly may be packaged with a filling aid and/or with a filling syringe. In some embodiments, the fill adapter may be packaged with a predetermined number of filling aids and/or with a predetermined number of filling syringes, e.g., 1 fill adapter packaged with 10 filling aids and 10 disposable housing assemblies. These numbers are examples only, and in various embodiments, the numbers may vary including being higher or lower than the examples given.

Referring now also to FIGS. 21A - 21F, another embodiment of the filling aid 9100 is shown. In this embodiment, the filling aid 9100 is configured to receive a filling syringe (not shown, shown as 9132 in FIGS. 24A- 24D and FIGS. 26A-26C), which includes a filling needle (not shown, shown as 9134 in FIG. 24A) attached to the filling syringe 9132. The filling aid 9100 in this embodiment includes a filling syringe holder 9102, which includes a filling needle cradle portion 9106 and a frame portion 9108. In various embodiments, the frame portion 9108 maintains the filling syringe such that it is attached to the filling aid 9100 and is not pulled out of the filling aid 9100. In some embodiments, the frame portion 9108 includes a half moon portion 9110 that accommodates the sliding portion of the filling syringe and also, provides a stop for the filling syringe such that if a user applies force in a direction opposite from the filling needle cradle portion 9106, the filling syringe will not be pulled out from the filling aid 9100. However, in various other embodiments, this portion may be shaped differently and some embodiments of the frame portion 9108 may not include a half moon (or differently shaped) portion 9110. In some embodiments, the filling syringe holder 9102 also include grips 9112, 9114 which, in various embodiments, may be shaped to ergonomically accommodate a portion of a user's thumb and forefinger. Some embodiments of the grips 9112, 9114 may include gripping features 9116 that may provide better traction for gripping the grips 9112, 9114. In various other embodiments, different features may be used, which may include, but is not limited to, ribs, raised circular features, embossed features and / or any type of feature in any shape and any quantity desired.

In some embodiments, the filling aid 9100 also includes a locking portion 9104 which may include one or more of the features described above with respect to various embodiments of the needle housing. In various embodiments, the filing syringe holder 9106 is slidably attached to the locking portion 9104, wherein, in various embodiments, the locking portion 9104 includes a groove feature 9130 on each side of the locking portion 9104 which accommodates a tongue feature 9128 from the filling needle cradle portion 9106 of the filling syringe holder 9102. These features work together to maintain the orientation of the locking portion 9104 with respect to the filling syringe holder 9102 and also, to provide a slidable relationship between the locking portion 9104 and the filling syringe holder 9102.

In the embodiments shown in FIGS. 21A - 23B, the locking portion 9104 includes locking features 9118 which interact with the filling syringe holder 9102 to lock the filling syringe holder 9102 either in a starting or filling position, with respect to the locking portion 9104. The filling syringe holder 9102 may be unlocked from its position by attaching the filling aid 9100 onto the filling aid base 9142. As the locking portion 9104 is attached to the filling aid base 9142, the locking features 9118 move to an unlocked position and the filling syringe holder 9102 may slide towards the filling aid base 9142. This occurs when the locking features 9118 are pushed out by the filling aid base 9142, which allows the filling syringe holder 9102 to become unlocked and slide with respect to the locking portion 9104.

Still referring to FIGS. 21A-23B, in various embodiments, the filling syringe holder 9102 includes a filling syringe cradle portion 9106 which includes filling needle cradle ribs 9124 which capture the filling syringe 9132 and maintain the filling syringe 9132 in an attached position with respect to the filling needle cradle 9106.

In various embodiments, the filling needle cradle 9106 also includes a stop feature 9126 which limits the distance the filling syringe 9132 may travel within the filling needle cradle 9016. However, the filling needle 9134 itself may travel through the stop feature 9126.

In various embodiments, once the filling syringe 9132 is attached to the filling aid 9100, the filling needle 9134 is attached to the end of the filling syringe 9132. In some embodiments, attaching the filling needle 9134 to the filling syringe 9132 may be done using a rotating motion. In some embodiments, the filling needle 9134 is attached to the filling syringe 9132 using a lure connection and is pressed onto the filling syringe 9132. However, in various other embodiments, any mechanism for attachment of a filling needle 9134 to a filling syringe 9132 may be used. In some embodiments, the filling needle 9134 is already attached to the filling syringe 9132.

Still referring to FIGS. 21A-23B, in various embodiments, the locking portion 9104 includes one or more tab features 9120. In various embodiments, the locking portion 9104 may include four tab features 9120 (two of which are shown in FIGS. 23A-23B). The tab features 9120 interact with either a vial (shown as 9136 in FIG. 24A) or a filling aid base 9142 and grip either the vial or filling base to secure the filling aid 9100 onto either the vial or the filling base. In various embodiments, the locking portion 9104 includes a syringe support 9122 which, in various embodiments, supports the filling syringe and, together with the frame portion 9108 of the filling syringe holder 9102, maintains the position of the filling syringe with respect to the filling aid 9100. These features prevent the filling syringe from moving with respect to the filling aid 9100 and therefore, maintain alignment of the filling needle 9134 with respect to the septum in the disposable housing assembly 9002.

Referring now also to FIGS. 24A- 24D, in practice, the filling syringe 9132 is attached to the filling aid 9100. In some embodiments, the filling needle 9134 may be attached to the filling syringe 9132, however, in some embodiments, the filling needle 9134 may have already been attached to the filling syringe 9132, for example, at manufacture. The cap (not shown) of the filling needle 9134 may then be removed. Next, the filling aid 9100 is attached to a vial of fluid 9136 by using the tabs features 9120 of the locking portion 9104. The vial of fluid may contain e.g., therapeutic fluid, e.g., insulin. The filling needle 9134 may penetrate the septum 9138 and the fluid may flow from the vial 9136 to the filling syringe 9132 by way of the filling needle 9134. Once a desired volume of fluid flows from the vial 9136 to the filling syringe 9132, the vial 9136 may be removed from the locking portion 9104, e.g., by exerting opposing force onto the locking housing 9104 and the vial 9136 so as to disconnect the tabs features 9120 of the locking portion 9104 from the vial 9136.

These embodiments may be advantageous, beneficial and/or desirable for many reasons including that the filling needle 9134 remains inside the locking portion 9104 while the filling syringe 9132 is being filled by the fluid from the vial 9136. Therefore, the likelihood of unintentional needle pricks and/or contamination is minimized and/or reduced. Thus, in various embodiments, the filling syringe holder 9102 remains in the starting and/or locked position while the filling aid 9100 is attached to a vial 9136.

Although the embodiments are described above with respect to a "vial" in some embodiments, the filling aid 9100 may be used in conjunction with any source of fluid, which may include, but is not limited to, a bag of fluid.

Referring now also to FIGS. 25A-25C, in various embodiments, once the filling syringe 9132 is removed from the vial 9136, the filling syringe 9132, still attached to the filling aid 9100 may then be connected to the filling aid base 9142 of the fill adapter 9140. The fill adapter 9140 may be connected to a disposable housing assembly 9002 (which, in various embodiments, includes a tubing and a cannula connection or a cannula at the end of the tubing). The fill adapter 9140 may be any one of the various embodiments of fill adapters described and shown herein. Further, in various embodiments, the fill adapter 9140 may be modified to include the embodiment of the filling aid base 9142 shown in FIGS. 25A-25C. In some embodiments, connection of the filling aid 9100 to the filling aid base 9140 may be made by sliding the filling aid 9100 over the filling aid base 9142. Thus, in various embodiments, the filling aid base 9142 may include a smaller diameter than the filling aid 9100 such that the filling aid base 9142 may be received by the filling aid 9100.

In some embodiments, the connection between the filling aid 9100 and the filling aid base 9142 may include the filling aid 9100 sliding onto the filling aid base 9142. The tab features 9120 slide over a groove on the filling aid base 9142. The filling aid base then interacts with the locking features 9118 and the locking features 9118 are moved to their unlocked position. The filling syringe holder 9102 then slides over the locking portion 9104 and towards the filling aid base 9142. At this point, the filling aid 9100 is attached or locked to the filling aid base 9142 such that the filling aid 9100 may not be removed from the filling aid base 9142 unless the filling aid 9100 is pulled back from the filling aid base 9142. Therefore, in some embodiments, the filling aid base 9142 may remain connected the filling aid 9100 while the disposable housing assembly 9002 reservoir 908 is being filled, but may be removed from each other once the filling is completed. Thus, in some embodiments, the filling aid 9100 may include a locked position with respect to the filling aid base 9142 and may include an unlocked position with respect to the filling aid base 9142.

In some embodiments, when the filling aid 9100 is connected to the filling aid base 9142, the filling needle cradle portion 9106 may be in a starting position. The starting position means that the filling needle 9134 remains fully inside the filling needle cradle portion 9106 and therefore, although the filling aid 9100 is attached to the filling needle base 9142, the filling needle 9134 has not pierced the septum of the reservoir 908. Referring now also to FIG. 954B, the filling needle cradle portion 9106 is in the starting position.

In various embodiments, when the filling needle cradle portion 9106 is in the starting position, the locking features 9118 are in the starting position and the relationship between the locking features 9118 and the filling needle cradle portion 9106 is such that the locking features 9118 anchor the filling needle cradle portion 9106 in the starting position.

As the locking portion 9104 is attached to the filling aid base 9142, to unlock the locking features 9118, the filling aid base 9142 interacts with the locking features 9118 such that they are moved to the unlocked position. The filling needle cradle portion 9106 may then slide towards the filling aid base 9142, sliding with respect to the locking portion 9104. Thus, the filling needle cradle portion 9106 is prevented from sliding with respect to the locking portion 9104 until and unless the locking portion 9104 is attached to the filling aid base 9142. Therefore, the filling needle 9134 is maintained within the filling needle cradle portion 9106 until the filling aid 9100 is attached to the filling aid base 9142. This embodiment may be beneficial for many reasons, including, but not limited to, prevention of needle contamination and/or damage and / or prevention of unintentional needle sticks, etc.

In various embodiments, to advance the filling needle 9134 towards the septum of the disposable housing assembly 9002, the filling needle cradle portion 9106 needs to be unlocked with respect to the locking portion 9104. This, in various embodiments, advances the filling needle cradle portion 9106 towards the septum such that the filling needle cradle portion 9106 is in the filling position. In this position, the filling needle 9134 pierces the septum of the reservoir 908 of the disposable housing assembly 9002.

In various embodiments locking the filling aid 9100 from movement with respect to the filling aid base 9142 when the filling needle cradle portion 9106 is in the filling position may be desirable for many reasons, including, but not limited to, once the filling needle 9134 pierces the septum, movement or rotation of the filling aid 9100 with respect to the filing aid base 9142 may cause damage to the septum and/or the reservoir and/or cause leakage.

Once the filling needle 9134 pierces the septum of the disposable housing assembly 9002, the plunger portion of the filling syringe 9132 may be advanced to provide flow of fluid from the filling syringe 9132 to the reservoir 908 of the disposable housing assembly 9002.

In various embodiments, the disposable housing assembly 9002 may have a tubing assembly 9090 and/or a tubing assembly 9090 connected to a cannula assembly 9092 (which, in some embodiments, may be a connection to a cannula assembly) attached.

In various embodiments, the fill adapter may be a reusable portion and the filling aid and filling syringe may be disposable/limited (e.g., one time) use portions. In some embodiments, the disposable housing assembly may be packaged with a filling aid and/or with a filling syringe. In some embodiments, the fill adapter may be packaged with a predetermined number of filling aids and/or with a predetermined number of filling syringes, e.g., 1 fill adapter packaged with 10 filling aids and 10 disposable housing assemblies. These numbers are examples only, and in various embodiments, the numbers may vary including being higher or lower than the examples given. In some embodiments, the filling aid 9100 may be packaged with the filling syringe 9132 and the filling syringe 9132 may be attached to the filling 9100. In some embodiments, the filling needle 9134 may be attached to the filling syringe 9132 in packaging and in some embodiments, the filling needle 9134 may not be attached to the filling syringe 9132 in packaging however, in some embodiments of this embodiment, the filling needle 9134 may be provided in the same package as the filling syringe 9132 and/or the filling aid 9100.

Referring now also to FIGS. 27A-27B, another embodiment of a fill adapter 9144 is shown. In this embodiment, the fill adapter 9144 includes a single piece frame which, in some embodiments, may be molded. The fill adapter 9144 frame includes one or more button assemblies 9148 corresponding to apertures in the disposable housing assembly 9002. In various embodiments, when fill adapter 9144 is releasably engaged with disposable housing assembly 9002, buttons assemblies 9148 may be aligned with apertures. Button assemblies 9148 may be, for example, members attached to a button assembly actuator 9146, capable of being depressed. When fill adapter 9144 is releasably engaged with disposable housing assembly 9002, one or more of button assemblies 9148 may be depressed, and may correspondingly be displaced through a respective one of apertures into reservoir 908, causing an attendant reduction in the volume of reservoir 908.

In various embodiments, the fill adapter 9144 may include one or more button assemblies and the disposable housing assembly may include one or more corresponding apertures. In various embodiments, the number, size, distribution and shape of the one or more button assemblies and the one or more apertures may be different than as shown herein. For example, in some embodiments, the button assemblies may be wider, may be round, may be square or may be thicker. Likewise, the corresponding aperture may accommodate the various embodiments of the button assemblies. In some embodiments, it may be desirable to vary the distribution, number, size and shape of the button assemblies, and correspondence apertures, to accommodate the volume of fluid that is anticipated to be filled in the reservoir.

In some embodiments, the button assemblies 9148 are actuated by at least one button assembly actuator 9146 which is actuated by applying a downward force upon the button assembly actuator 9146. In some embodiments, each of the at least one button assemblies 9148 may be separately actuated by a dedicated button assembly actuator. The button assembly actuator 9146 may be any size desired, but in some embodiments, may be as shown in FIGS. 27A- 27C. As shown in, for example, FIG. 27A, the button assembly actuator 9146 may include visible indicators, for example, a fluid drop icon, other icons, for example, a "press" icon, to indicate the method of actuation. In some embodiments, the button assembly actuator 9146 may include a depression and/or ergonomic finger and/or thumb accommodation.

In the exemplary embodiment of this embodiment of the fill adapter 9144, the fill adapter 9144 includes a housing, the button assembly actuator 9146 springingly attached to the housing. The fill adapter 9144 may include a pump chamber plunger 9150 shown in, for example, FIG. 27D, on the underside of the fill adapter 9144 housing. In various embodiments, the pump chamber plunger 9150 may be actuated together with the button assemblies 9148 via the button assembly actuator 9146.

Referring now also to FIGS. 28A-28B, upon coupling the fill adapter 9144 to the disposable housing assembly 9002, the reservoir 908 may be filled using a filling syringe 9132. Any syringe known in the art may be used, however, in the exemplary embodiments, any syringe having a size and shape to be accommodated by the filling aid 6004 may be used, including, but not limited to, a 3cc/mL TERUMO SYRINGE without needle, made by TERUMO Europe, Belgium, together with a Becton Dickinson 26G1/2 PRECISIONGLIDE Needle, made by Becton Dickinson & Co., Franklin Lakes, New Jersey, U.S.A., however, in various embodiments, the filling syringe 9132 may be a syringe made by another manufacture and/or at a larger or smaller size. Fill adapter 9144 may include an outside portion and a cavity portion. The cavity portion is the portion that mates with the disposable housing assembly 9002. The fill adapter 9144 may include locking tabs 9152 that may be configured to engage the disposable housing assembly 9002 in a manner such that the disposable housing assembly 9002 may be held in the cavity portion of the fill adapter 9144. Accordingly, fill adapter 9144 may be releasably engaged with disposable housing assembly 9002 by aligning fill adapter 9144 with disposable housing assembly 9002 and applying force such that force is applied to the disposable housing assembly 9002 relative to the fill adapter 9144 or vice versa. In some embodiments, the fill adapter 9144 may attach to the disposable housing assembly 9002 by rotating the fill adapter 9144 with respect to the disposable housing assembly 9002 in a similar fashion as described above with respect to attaching the reusable pump assembly to the disposable housing assembly.

In various embodiments, the fill adapter 9144 includes a filling aid base 9142.

Referring now also to FIGS. 29A-29C, another embodiment of a fill adapter 9244 is shown. In this embodiment, the fill adapter 9244 includes a single piece frame which, in some embodiments, may be molded. The fill adapter 9244 frame includes one or more button assemblies 9248 corresponding to apertures in the disposable housing assembly (not shown). In various embodiments, when fill adapter 9244 is releasably engaged with disposable housing assembly 9002, buttons assemblies 9248 may be aligned with the apertures. Button assemblies 9248 may be, for example, members attached to a button assembly actuator 9246, capable of being depressed. When fill adapter 9244 is releasably engaged with disposable housing assembly 9002, one or more of button assemblies 9248 may be depressed, and may correspondingly be displaced through a respective one of the apertures into the reservoir, causing an attendant reduction in the volume of the reservoir.

In various embodiments, the fill adapter 9244 may include one or more button assemblies and the disposable housing assembly may include one or more corresponding apertures. In various embodiments, the number, size, distribution and shape of the one or more button assemblies and the one or more apertures may be different than as shown herein. For example, in some embodiments, the button assemblies may be wide, narrow, round, square. Likewise, the corresponding aperture may accommodate the various embodiments of the button assemblies. In some embodiments, it may be desirable to vary the distribution, number, size and shape of the button assemblies, and correspondence apertures, to accommodate the volume of fluid that is anticipated to be filled in the reservoir.

In some embodiments, in various embodiments the button assemblies 9248 are actuated by at least one button assembly actuator 9246 which is actuated by applying a downward force upon the button assembly actuator 9246. In some embodiments, each of the at least one button assemblies 9248 may be separately actuated by a dedicated button assembly actuator. The button assembly actuator 9246 may be any size desired, but in some embodiments, may be as shown in FIGS. 29A- C. As shown in, for example, FIG. 29A, the button assembly actuator 9246 may include visible indicators, for example, a fluid drop icon, other icons, for example, a "press" icon, to indicate the method of actuation. In some embodiments, the button assembly actuator 9246 may include a depression and/or ergonomic finger and/or thumb accommodation.

In the exemplary embodiment of this embodiment of the fill adapter 9244, the fill adapter 9244 includes a housing, the button assembly actuator 9246 springingly attached to the housing. The fill adapter 9244 may include a pump chamber plunger 9250 shown in, for example, FIG. 6C, on the underside of the fill adapter 9244 housing. In various embodiments, the pump chamber plunger 9250 may be actuated together with the button assemblies 9248 via the button assembly actuator 9246.

Upon coupling the fill adapter 9244 to a disposable housing assembly 9002, the reservoir 908 may be filled using a filling syringe. Any syringe known in the art may be used, however, in the exemplary embodiments, any syringe having a size and shape to be accommodated by the filling aid 6004 may be used, including, but not limited to, a 3cc/mL TERUMO SYRINGE without needle, made by TERUMO Europe, Belgium, together with a Becton Dickinson 26G1/2 PRECISIONGLIDE Needle, made by Becton Dickinson & Co., Franklin Lakes, New Jersey, U.S.A., however, in various embodiments, the filling syringe may be a syringe made by another manufacture and/or at a larger or smaller size. Fill adapter 9244 may include an outside portion and a cavity portion. The cavity portion is the portion that mates with the disposable housing assembly 9002. The fill adapter 9244 may include a locking tabs 9252 that may be configured to engage the disposable housing assembly 9002 in a manner such that the disposable housing assembly 9002 may be held in the cavity portion of the fill adapter 9244. Accordingly, fill adapter 9244 may be releasably engaged with disposable housing assembly 9002 by aligning fill adapter 9244 with disposable housing assembly 9002 and applying force such that force is applied to the disposable housing assembly 9002 relative to the fill adapter 9244 or vice versa. In some embodiments, the fill adapter 9244 may attach to the disposable housing assembly 9002 by rotating the fill adapter 9244 with respect to the disposable housing assembly 9002 in a similar fashion as described above with respect to attaching the reusable pump assembly to the disposable housing assembly.

In some embodiments, the fill adapter 9144 includes a filling aid base 9242. In some embodiments, the filling aid base 9242 may include a tapered area 9254 that may be beneficial/desirable for many reasons, including, but not limited to, controlling the entry of a filling needle such that the filling needle is maintained in an aligned position while filling the disposable housing assembly 9002 the reservoir. In various embodiments, the tapered area 9254 may be sized and shaped as shown or the size and / or the shape may vary in some embodiments. Any one of more of the various embodiments of the filling aid base shown herein may also include a tapered area as shown and describe herein.

In various embodiments, these methods may be used with respect to any device and/or medical device and/or any controller and/or remote controller for any device and/or medical device and/or any device used in conjunction with or in association with any device and/or medical device.

A number of embodiments have been described. Nevertheless, it will be understood that various modifications may be made. Accordingly, other embodiments are within the scope of the following claims.

While the principles of the invention have been described herein, it is to be understood by those skilled in the art that this description is made only by way of example and not as a limitation as to the scope of the invention. Other embodiments are contemplated within the scope of the present invention in addition to the exemplary embodiments shown and described herein. Modifications and substitutions by one of ordinary skill in the art are considered to be within the scope of the present invention.

## Claims

1. An infusion pump system comprising:
a reusable housing assembly (6004) comprising a pump actuator assembly;
a disposable housing assembly (26012) comprising:
a reservoir;
a tab portion (26116), the tab portion comprising a female latching feature (26114); and
an exit, the exit fluidly connected to the reservoir; and
a fluid connector (26100) comprising:
a body portion (26108);
a plug portion (26102) comprising a fluid path,
a catch feature (26118) located on a first end of the body portion and configured to interact with the disposable housing assembly;
a male latching feature (26110) located on a second end of the body portion, the male latching feature configured to interact and lock onto the female latching feature; and
a tubing (26134), a first end of the tubing fluidly connected to the fluid path of the plug portion;
wherein the plug of the connector forms a luer connection with the exit of the disposable housing assembly and provides a fluid connection between the reservoir and the tubing,
wherein the reusable housing assembly is attachable to the disposable housing assembly,
wherein the pump actuator assembly pumps fluid from the reservoir to the tubing,
wherein the plug has an exit end portion and a tubing end portion; and
wherein the body portion comprises an opening for receiving the plug portion (26102) and a slot (26120) and the plug portion is slidably connected to the body portion and comprises a disc (26122), the disc configured to seat within the slot (26120) when the plug portion is in the opening of the body portion;
**characterized in that**
the exit end portion comprises a stop feature for limiting movement of the plug portion in one direction through the opening and the tubing end portion comprises a second stop feature for limiting movement of the plug portion in an opposite direction through the opening;
wherein force applied to the plug portion may overcome a threshold force and unseat the disc from the slot wherein the plug portion (26102) moves with respect to the body portion.

2. The infusion pump system of claim 1, wherein the reusable housing assembly (6004) further comprising a volume sensor assembly (148).

3. The infusion pump system of claim 1, wherein the second end of the tubing connected to a cannula assembly (9092).

4. The infusion pump system of claim 1, wherein the connector (26100) wherein the body portion (26108) comprises an indent (26124) wherein the indent configured to interact with a reusable portion of an infusion pump.

5. The infusion pump system of claim 1, wherein the reusable housing assembly (6004) further comprising a locking ring assembly.

6. The infusion pump system of claim 5, wherein the locking ring assembly further comprising a nub (808) comprising a spring actuated tab, the spring actuated tab interacts with the indent (26124) of the connector (26108) when the reusable housing assembly (6004) and disposable housing assembly (26012) are rotatably attached.

7. The infusion pump system of claim 6, wherein the spring actuated tab of the nub (808) interacts with the connector (26100) exerting compression onto the connector attachment to the exit of the disposable housing assembly (26012).

8. The infusion pump system of claim 1, wherein the body portion (26108) further comprising rib features.

9. The infusion pump system of claim 1, wherein the catch feature (26118) comprising a ramp.

10. The infusion pump system of claim 1, wherein the underside of the body portion (26108) comprising a core (26040).

11. The infusion pump system of claim 10, wherein the core (26040) comprising an identification tag.

12. The infusion pump system of claim 1, wherein the body portion (26108) comprising an identification tag.

13. The infusion pump system of claim 12, wherein the identification tag is an RFID tag.

14. The infusion pump system of claim 12, wherein the identification tag is a near-field communication readable RFID.

## Patentansprüche

1. Infusionspumpensystem, umfassend:
eine wiederverwendbare Gehäuseanordnung (6004), umfassend eine Pumpenbetätigungsanordnung;
eine Einweg-Gehäuseanordnung (26012), umfassend:
einen Behälter;
einen Laschenabschnitt (26116), der Laschenabschnitt umfassend Buchsenverriegelungselement (26114); und
einen Ausgang, wobei der Ausgang fluidisch mit dem Behälter verbunden ist, und
einen Fluidverbinder (26100), umfassend:
einen Körperabschnitt (26108);
einen Stopfenabschnitt (26102), umfassend einen Fluidweg,
ein Fangelement (26118), das sich an einem ersten Ende des Körperabschnitts befindet und konfiguriert ist, um mit der Einweg-Gehäuseanordnung zusammenzuwirken;
ein Steckerverriegelungselement (26110), das an einem zweiten Ende des Körperabschnitts angeordnet ist, wobei das Steckerverriegelungselement konfiguriert ist, um mit dem Buchsenverriegelungselement zusammenzuwirken und in dieses einzurasten; und
einen Schlauch (26134), wobei ein erstes Ende des Schlauchs fluidisch mit dem Fluidweg des Stopfenabschnitts verbunden ist;
wobei der Stopfen des Verbinders eine Luer-Verbindung mit dem Ausgang der Einweg-Gehäuseanordnung bildet und eine Fluidverbindung zwischen dem Behälter und dem Schlauch herstellt,
wobei die wiederverwendbare Gehäuseanordnung an der Einweg-Gehäuseanordnung angebracht werden kann,
wobei die Pumpenbetätigungsanordnung Fluid aus dem Behälter in den Schlauch pumpt,
wobei der Stopfen einen Ausgangsendabschnitt und einen Schlauchendabschnitt aufweist; und
wobei
der Körperabschnitt eine Öffnung zum Aufnehmen des Stopfenabschnitts (26102) und einen Schlitz (26120) umfasst und der Stopfenabschnitt gleitend mit dem Körperabschnitt verbunden ist und eine Scheibe (26122) umfasst, wobei die Scheibe konfiguriert ist, um in dem Schlitz (26120) zu sitzen, wenn der Stopfenabschnitt in der Öffnung des Körperabschnitts ist;
**dadurch gekennzeichnet, dass**,
der Ausgangsendabschnitt ein Anschlagelement zum Begrenzen einer Bewegung des Stopfenabschnitts in einer Richtung durch die Öffnung umfasst und der Schlauchendabschnitt ein zweites Anschlagelement zum Begrenzen einer Bewegung des Stopfenabschnitts in einer entgegengesetzten Richtung durch die Öffnung umfasst;
wobei eine auf den Stopfenabschnitt ausgeübte Kraft eine Schwellenwertkraft überwinden und die Scheibe aus dem Schlitz lösen kann, wobei sich der Stopfenabschnitt (26102) in Bezug auf den Körperabschnitt bewegt.

2. Infusionspumpensystem nach Anspruch 1, die wiederverwendbare Gehäuseanordnung (6004) ferner umfassend eine Volumensensoranordnung (148).

3. Infusionspumpensystem nach Anspruch 1, wobei das zweite Ende des Schlauchs mit einer Kanülenanordnung (9092) verbunden ist.

4. Infusionspumpensystem nach Anspruch 1, wobei der Verbinder (26100), dessen Körperabschnitt (26108) eine Vertiefung (26124) aufweist, wobei die Vertiefung konfiguriert ist, um mit einem wiederverwendbaren Teil einer Infusionspumpe zusammenzuwirken.

5. Infusionspumpensystem nach Anspruch 1, die wiederverwendbare Gehäuseanordnung (6004) ferner umfassend eine Sicherungsringanordnung.

6. Infusionspumpensystem nach Anspruch 5, die Sicherungsringanordnung ferner umfassend einen Noppen (808), der eine federbetätigte Lasche aufweist, wobei die federbetätigte Lasche mit der Vertiefung (26124) des Verbinders (26108) zusammenwirkt, wenn die wiederverwendbare Gehäuseanordnung (6004) und die Einweg-Gehäuseanordnung (26012) drehbar angebracht sind.

7. Infusionspumpensystem nach Anspruch 6, wobei die federbetätigte Lasche des Noppens (808) mit dem Verbinder (26100) zusammenwirkt und am Ausgang der Einweg-Gehäuseanordnung (26012) Druck auf die Verbinderbefestigung ausübt.

8. Infusionspumpensystem nach Anspruch 1, der Körperabschnitt (26108) ferner umfassend Rippenelemente.

9. Infusionspumpensystem nach Anspruch 1, das Fangelement (26118) umfassend eine Rampe.

10. Infusionspumpensystem nach Anspruch 1, die Unterseite des Körperabschnitts (26108) umfassend einen Kern (26040).

11. Infusionspumpensystem nach Anspruch 10, der Kern (26040) umfassend ein Identifikationsanhänger.

12. Infusionspumpensystem nach Anspruch 1, der Körperabschnitt (26108) umfassend ein Identifikationsanhänger.

13. Infusionspumpensystem nach Anspruch 12, wobei der Identifikationsanhänger ein RFIDanhänger ist.

14. Infusionspumpensystem nach Anspruch 12, wobei der Identifikationsanhänger eine durch Nahfeldkommunikation lesbare RFID ist.

## Revendications

1. Système de pompe à perfusion comprenant :
un ensemble boîtier réutilisable (6004) comprenant un ensemble actionneur de pompe ;
un ensemble boîtier jetable (26012) comprenant :
un réservoir ;
une partie languette (26116), la partie languette comprenant un élément de verrouillage femelle (26114) ; et
une sortie, la sortie étant raccordée fluidiquement au réservoir ; et
un raccord fluidique (26100) comprenant :
une partie corps (26108) ;
une partie bouchon (26102) comprenant un trajet de fluide,
un élément loquet (26118) situé sur une première extrémité de la partie corps et conçu pour interagir avec l'ensemble boîtier jetable ;
un élément de verrouillage mâle (26110) situé sur la seconde extrémité de la partie corps, l'élément de verrouillage mâle étant conçu pour interagir et se verrouiller sur l'élément de verrouillage femelle ; et
un tube (26134), une première extrémité du tube étant raccordée fluidiquement au trajet de fluide de la partie bouchon ;
ledit bouchon du raccord formant un raccordement Luer avec à la sortie de l'ensemble boîtier jetable et fournissant un raccordement fluidique entre le réservoir et le tube,
ledit ensemble boîtier réutilisable pouvant être fixé à l'ensemble boîtier jetable,
ledit ensemble actionneur de pompe pompant le fluide du réservoir vers le tube, ledit bouchon comportant une partie d'extrémité de sortie et une partie d'extrémité de tube ; et
ladite partie corps comprenant une ouverture destinée à recevoir la partie bouchon (26102) et une fente (26120) et ladite partie bouchon étant raccordée de manière coulissante à la partie corps et
comprenant un disque (26122), le disque étant conçu pour se loger dans la fente (26120) lorsque la partie bouchon est dans l'ouverture de la partie corps ;
**caractérisé en ce que**
la partie d'extrémité de sortie comprend un élément d'arrêt pour limiter le mouvement de la partie bouchon dans une direction à travers l'ouverture et la partie d'extrémité de tube comprend un second élément d'arrêt pour limiter le mouvement de la partie bouchon dans une direction opposée à travers l'ouverture ;
ladite force appliquée à la partie bouchon pouvant surmonter une force seuil et déloger le disque de la fente, ladite partie bouchon (26102) se déplaçant par rapport à la partie corps.

2. Système de pompe à perfusion selon la revendication 1, ledit ensemble boîtier réutilisable (6004) comprenant en outre un ensemble capteur de volume (148).

3. Système de pompe à perfusion selon la revendication 1, ladite seconde extrémité du tube étant raccordée à un ensemble canule (9092).

4. Système de pompe à perfusion selon la revendication 1, ledit raccord (26100), ladite partie de corps (26108) comprenant un renfoncement (26124), ledit renfoncement étant conçu pour interagir avec une partie réutilisable d'une pompe à perfusion.

5. Système de pompe à perfusion selon la revendication 1, ledit ensemble boîtier réutilisable (6004) comprenant en outre un ensemble bague de verrouillage.

6. Système de pompe à perfusion selon la revendication 5, ledit ensemble bague de verrouillage comprenant en outre une bosse (808) comprenant une languette actionnée par ressort, ladite languette actionnée par ressort interagissant avec le renfoncement (26124) du raccord (26108) lorsque l'ensemble boîtier réutilisable (6004) et l'ensemble boîtier jetable (26012) sont fixés de manière rotative.

7. Système de pompe à perfusion selon la revendication 6, ladite languette actionnée par ressort de la bosse (808) interagissant avec le raccord (26100) exerçant une compression sur la fixation de raccord jusqu'à la sortie de l'ensemble boîtier jetable (26012).

8. Système de pompe à perfusion selon la revendication 1, ladite partie corps (26108) comprenant en outre des éléments nervures.

9. Système de pompe à perfusion selon la revendication 1, ledit élément loquet (26118) comprenant une rampe.

10. Système de pompe à perfusion selon la revendication 1, ladite face inférieure de la partie corps (26108) comprenant un noyau (26040).

11. Système de pompe à perfusion selon la revendication 10, ledit noyau (26040) comprenant une étiquette d'identification.

12. Système de pompe à perfusion selon la revendication 1, ladite partie corps (26108) comprenant une étiquette d'identification.

13. Système de pompe à perfusion selon la revendication 12, ladite étiquette d'identification étant une étiquette RFID.

14. Système de pompe à perfusion selon la revendication 12, ladite étiquette d'identification étant une RFID lisible par communication en champ proche.
